(19)

Europäisches
Patentamt
European
Patent Office
Office européen
des brevets

(11)     **EP 1 890 595 B1**

(12)     **EUROPEAN PATENT SPECIFICATION**

(45) Date of publication and mention
of the grant of the patent:
**07.03.2012   Bulletin 2012/10**

(51) Int Cl.:
*A61B 5/044* (2006.01)          *A61B 5/04* (2006.01)
*A61B 5/0402* (2006.01)         *A61B 5/0452* (2006.01)
*G06K 9/00* (2006.01)

(21) Application number: **06744310.1**

(22) Date of filing: **10.05.2006**

(86) International application number:
**PCT/GB2006/050099**

(87) International publication number:
**WO 2006/126020 (30.11.2006 Gazette 2006/48)**

(54) **MEDICAL DATA SIGNAL PROCESSING SYSTEMS**

SYSTEME ZUR VERARBEITUNG VON MEDIZINISCHEN DATENSIGNALEN

SYSTEMES MEDICAUX DE TRAITEMENT DE SIGNAUX DE DONNEES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **24.05.2005   GB 0510446
03.06.2005   US 687598 P**

(43) Date of publication of application:
**27.02.2008   Bulletin 2008/09**

(73) Proprietor: **ANGLIA POLYTECHNIC UNIVERSITY
HIGHER EDUCATION
CORPORATION
Chelmsford, Essex CM1 1SQ (GB)**

(72) Inventors:
• **AL-HASSANI, Aimen
Brentwood,
Essex CM13 2PA (GB)**
• **ZIZZO, Claudio
Chelmsford,
Essex CM1 6BF (GB)**

(74) Representative: **Martin, Philip John
Marks & Clerk LLP
62-68 Hills Road
Cambridge
CB2 1LA (GB)**

(56) References cited:
**EP-A- 1 488 739        WO-A-01/67951
WO-A-92/05831        US-A1- 2003 167 013
US-B1- 6 694 178**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This invention relates to medical data signal processing apparatus, methods, and computer program code, and in particular to systems for processing ECG (Electrocardio graph) data.

Heart and circulatory disease is the UK's biggest killer - for example in 2002 accounting for 39 percent of deaths in the UK. In Europe cardiovascular disease, the main form of which is coronary heart disease or ischemia, accounts for nearly 2 million deaths annually. Cost of health care across Europe runs into billions of pounds a year.

When a patient is admitted to hospital they will generally be put under observation. Typically the patient is monitored by non-specialist health professionals such as nurses, with specialist clinicians (doctors or consultants) making occasional visits. Typically ECG signals from the patient are presented on a video display and/or as one or more traces on paper, but these signals are difficult to interpret. Commercial heart monitoring systems generally monitor one or three ECG signals (leads) although for particularly severe conditions a twelve lead/electrode configuration is employed to acquire the ECG signals. Even where one or more channels is present typically only one channel is monitored because of the difficulty of interpretation. To detect potential ischemia the ST level of an ECG trace is examined (this is a difference in voltage between two parts of the cardiac signal, as discussed further below) but it is often difficult to accurately determine an ST level. It is still more difficult for a non-specialist to determine whether or not to call in a specialist, and this decision is subject to human error.

There is therefore a need for a tool to assist a non-specialist in deciding whether or not call in a specialist doctor or consultant. By contrast prior research has focussed on devices which require the expertise of a specialist consultant to be used effectively (for example US 2005/00389352) and on the development of algorithms for the automatic detection of pathological heart conditions. A system for displaying ECG data as a 3D topographical model of a heart is described in US G,b94,178, but this still requires expert interpretation. Further background prior art can be found in:

Packing and Clinical Electrophysiology, Vol. 21, No. 6, June 1998 (USA), L Gepstein & S J Evans, "Electroanatomical mapping of the heart: basic concepts and implications for the treatment of cardiac arrhythmias", pages 1268-1278.

IEEE Transactions on Medical Imaging, Vol. 21, No. 9, September 2002, B Tilg et al, "Model-Based Imaging of Cardiac Electrical Excitations in Humans", pages 1031-1039.

US 4,846,190 A (John) see abstract, cols. 3, 4, 12, 14, & 15 and figs. 3, 6, 8, and 9.

US 4,579,125 A (Strobl et al) see abstract, cols. 3, 4 and 8-10 and fig. 4B.

US 4,862,359 A (Trivedi et al) see abstract, cols. 11 & 12, and figs. 5 & 7.

US 6,694,178 B1 (Soula et al) see abstract, cols, 5 & 10, and fig. 14.

JP 05137702 A (Sharp) see EPODOC and PAJ abstracts.

European patent application EP 1488739, published 2004-12-22, inventors Nagata Shinya et al. and applicant Dainippon Pharmaceutical Co, describes that an electrocardiogram (ECG) chart device and method is capable of easily assisting with the diagnosis of heart disease. Hexagonal radar charts displayed on a screen act as indicators of feature values corresponding to data obtained from each of 12 electrode leads and correlated with the related portions of the heart. For example, a (V1, V2) lead is an indicator of a right ventricle. Each of the radar charts is schematically arranged to correspond with the related portion of the heart. Each vertex of the hexagonal radar charts acts as an indicator of the recognized value.

US patent application Ups2003167013, published 2003-09-04, inventors and applicant Anatoly Sula, et al., discloses using standard ECG signals that are analyzed through an electrodynamical model of electrical field generation by the heart, which gives information from ECG fluctuations. It aims to allow a more detailed reconstruction of the heart's electrical processes and visualization of a precise anatomical picture of a dynamics of heart processes.

According to a first aspect of the invention, there is provided a medical signal processign system for processing medical signal data obtained from a human or animal heart, and displaying a graphical representation of the processed data for providing an ischemia onset alert, as defined in appended independent claim 1.

There may further be provided a medical image display system comprising the medical signal processing system of the first aspect.

According to a second aspect of the invention, there is provided a method of displaying medical data to indicate ischemia onset of a heart, as defined in appended independent claim 20.

Preferred embodiment are defined in the appended dependent claims.

These and other aspects of the present invention will now be further described, by way of example only, with reference to the accompanying figures in which:

Figure 1 shows a typical cardiac cycle signal;

Figure 2 shows positions on the heart wall to which electrode leads map, for a twelve lead ECG captured signal, on a 3D heart image;

Figures 3 shows, positions on the heart wall to which electrode leads map, for a twelve lead ECG captured signal on a 2D heart cross-section;

Figures 4a to 4c show a time series of 3D images of a heart and a superimposed graphical display of abnormalities;

Figure 5 shows a time series of images of a cross-sectional view of a heart and a superimposed graphical display of abnormalities;

Figure 6 shows an example of a user-interface display of an embodiment of a system according to the present invention;

Figure 7 shows a block diagram of an embodiment of a system according to the present invention;

Figure 8 shows a flow diagram of a signal conditioning and analysis procedure for the system of figure 7;

Figure 9 shows a flow diagram of a graphical display procedure for the system of figure 7;

Figure 10 shows a flow diagram of a 2D grid-based graphical abnormality display procedure for the procedure of figure 8; and

Figure 11 shows a block diagram of a further medical image display system according to an embodiment of the invention.

The following describes an embodiment of a medical signal processing system for processing medical signal data obtained from a human or animal body and relating to an organ in said body, and displaying a graphical representation of the processed data for providing an alert, the system comprising: a medical signal data input; a graphical display; and a signal processor coupled to said data input and to said display; and wherein said signal processor is configured to: input medical signal data associated with a spatial position in or on said organ; perform at least one measurement relating to said organ using said medical signal data to provide measurement data; determine from said measurement data whether said measurement is normal or abnormal; and display, on a graphical representation of said organ, a graphical indication of an abnormality at said spatial position with which said signal data is associated responsive an abnormal to said determination.

In embodiments of the system the graphically displayed information relates to one or more specific pathological conditions of the organ, for example in the case of a heart, ischemia, and because the information is of diagnostic relevance to the displayed organ it is easier for a non-specialist nurse or technician to identify whether or not a specialist opinion should be sought.

The medical signal data provided to the system may either come directly from the patient, for example from electrodes attached to the patient, or via an intermediate signal acquisition instrument. A measurement may be performed directly on the medical signal data or on data derived from the data, for example on an average signal. In embodiments the determination of whether a measurement is normal or abnormal may be made by comparing the measurement with a threshold (either absolute or relative/normalised) and/or one or more rules may be applied, for example to identify the presence of a pathological condition associated with the signal from an electrode.

Preferably a validation procedure is also applied when determining whether a measure is normal or abnormal, for example by applying a filter to determine whether, say, there is more than a threshold number of an abnormal events over normal events in a time window. Thus embodiments of the system also perform a time correlation analysis on the input data, to which the graphical abnormality display is responsive.

In some preferred embodiments the display only indicates when an abnormality in a measurement has been identified and optionally verified. The abnormality determination may categorise the measurement according to gravity or severity of then abnormality, and the graphical indication may then be coded, for example by colour, in response. Preferably the graphical representation of an abnormality is also responsive to measurement history. So that, for example, when a (validated) abnormality has been present in the past but is no longer present, this is shown on the display. In this way transient events may be detected without continuous monitoring. In embodiments the graphical representation of an abnormality also indicates the duration of the abnormality, for example by displaying a "problem" region (line or area) on the display which grows over time and/or with severity of the abnormality.

In preferred embodiments the input medical signal data comprises a plurality of signals associated with a corresponding plurality of spatial positions in or on the organ. For example where ECG signals are captured from a plurality of electrodes ("leads") these are generally located at standard positions so that the signal from each electrode may be mapped to a position on the heart wall. These positions are well known and tabulated for different numbers of leads. In such cases the graphical display preferably indicates, for each of the signals, the graphical representation of the abnormality at the relevant spatial position on the organ associated with the electrode (or other captured means) from which the signal is derived (although non-standard positions can also be mapped). Preferably the data is displayed graphically on a 2D or 3D representation of the organ, showing electrode positions and an abnormality in a signal from an electrode as a growing region of colour at the relevant position.

Where a plurality of signals is processed the graphical abnormality indication for one signal may be responsive to measurements made on one or more others of the signals. Thus, generally speaking, an indication of a more severe problem may be displayed when two or more input signals indicate an abnormality. In one particular embodiment a grid is constructed on which abnormal signals are indicated at positions on the organ to which the signals map, by increasing or decreasing the number of grid positions which are occupied by a graphical abnormality representation, optionally also changing the colour of each grid position. With this approach the graphical indication at any particular grid position may be responsive to the graphical indication at one or more neighbouring positions. This may be achieved by determining a value for each position on the grid from the normal/abnormal determination for each processed signal by performing a number of iterations, in each iteration the value of a grid point is determined from one or more of a current status/level of the point, the level/status of nearby or adjacent points, a history of the status/level of the point, a history of the status/level of nearby or adjacent points and optionally other parameters. Broadly speaking in embodiments this approach results in an area which spreads out from each signal spatial position over the displayed image of the organ, continuing to spread as the abnormal condition persists and contracting once again once the abnormal condition ceases. During the contracting phase a different colour may be employed to indicate a past abnormality. Where two growing regions meet they may mutually enhance one another to indicate greater severity.

In some preferred embodiments of the system the organ comprises a heart and the medical signal data one or more ECG signals. The results of processing these signals are displayed at corresponding spatial positions (generally standard positions) on a two-dimensional or three-dimensional graphical representation of the heart wall. For example a 2D view of the heart may comprise one or more of a vertical frontal plane and a pericardial plane through the heart. For a 2D representation an abnormal condition may be represented by a line the length of which grows or contracts according to the degree or duration of the abnormality; for a 3D view of the heart the abnormality may be represented by a growing or contracting region on the heart wall. In an alerting system for ischemia the measurement preferably comprises an ST level measurement (elevation or drop). This may be compared with a threshold (either absolute or percentage change from an isoelectric baseline) to determine whether the measurement is normal or abnormal and, optionally, to what degree.

Preferred embodiments of the system are configured for real-time processing of the medical signal data and corresponding graphical abnormality indication, to facilitate real-time patient monitoring. Preferably, however, the system also includes a data store to store one or more of the medical signal data, the measurement data, and the displayed graphical data to facilitate data replay and analysis, for example where it can be seen that a past abnormality has occurred but has been missed. Alternatively the system can be used offline only to post-process captured data, for example as a research tool.

Preferably the signal processor comprises a micro-processor and program memory storing processor control code so that the signal processor is configured by implementation of the code by the micro-processor. Broadly speaking the system may be implemented on any conventional data processing system such as an embedded PC or DSP (Digital Signal Processor). However where many signals are to be processed in substantially real-time it may be preferable to implement some or all of the signal processing in hardware for example using an ASIC (Application Specific Integrated Circuit) or FPGA (Field Programmable Gate Array).

Processor control code may be provided for configuring a signal processor to implement the system as described above. This code is generally provided on a data carrier such as a magnetic and/or optical disk programmed memory such as read-only memory, or on a data carrier such as an optical or electrical signal carrier. The code may comprise code in any conventional programming language, for example Visual Basic (trade mark) or C, or code for setting up or controlling an ASIC or FPGA, or code for a hardware description language such as VeriLog (trade mark) or VHDL. As the skilled person will appreciate such code may be distributed between a plurality of coupled components in communication with one another.

The following describes an embodiment ofa method of processing medical signal data obtained from a human or animal body and relating to an organ in said body and displaying a graphical representation of the processed data for providing an alert for calling a doctor or consultant, the system comprising: a medical signal data input; a graphical display; and a signal processor coupled to said data input and to said display; the method comprising: inputting medical signal data associated with a spatial position in or on said organ; performing at least one measurement relating to said organ using said medical signal data to provide measurement data; determining from said measurement data whether said measurement is normal or abnormal; and displaying, on a graphical representation of said organ, a graphical indication of an abnormality at said spatial position with which said signal data is associated responsive an abnormal to said determination.

Processor control code, in particular on a carrier, for example as described above, may be provided to implement the above method.

The following describes an embodiment of a medical image display system, the system comprising: an image capture system to capture an image of an internal organ of a patient; a medical signal processing system to capture medical signal data from said imaged organ of said patient and to process said captured medical signal data to (automatically) identify an abnormality; and an image display system coupled to said image capture system and to said medical image processing system, to display an image of said internal organ together with a graphical representation of said abnormality.

Preferably the internal organ comprises a heart of a human or animal and the captured medical data signal comprises ECG data. In embodiments the signal processing system is configured to process (analyse) the captured medical signal data by measurement of one or more features of the captured medical signal data and comparison with a reference such as a norm. Preferably the medical signal processing system is configured to identify one or both of an ischemic abnormality of the heart and a change in rhythm of the heart (arrhythmia). In some particularly preferred embodiments the image capture system comprises an x-ray fluoroscopy imaging system.

Some preferred embodiments of the system are particularly useful for angioplasty. During x-ray fluoroscopy imaging a contrast agent is injected. Typically a faint view of the heart is available, with a good view of the catheter, until injection of this contrast agent. It is desirable to monitor ECG signals during the procedure because a catheter can at least partially obstruct an artery, and this is not clearly visible on the x-ray image. In preferred embodiments of the system the graphical representation of the abnormality, either on a graphical representation of the organ or on the imaged organ itself, is displayed together with the image of the internal organ (heart). This facilitates monitoring of the ECG signals, potentially by the surgeon carrying out the operation. The graphical representation of the abnormality, preferably on a graphical representation of the organ, can either be displayed in proximity to the image of the organ, for example on side-by-side display screens or using a split screen display, or overlapping or otherwise fused by means of image fusion techniques.

Fusion of the two images may be performed in a relatively straightforward manner since an x-ray fluoroscopy (or other) imaging system generally provides X Y (and optionally Z) coordinates defining an image view. In many imaging systems the geometry of the system and the position of the patient relative to the system is defined and known. Additionally or alternatively this information may be derived from one or more fiducial marks in the imaged target and/or be adjusted by an operator control such as a joystick. Optionally the graphical representation of the abnormality (optionally on the graphical representation of the organ) may be altered to correspond with the view captured by the image capture system (and displayed on the display). Thus the two images (abnormality and imaged organ) may track one another.

Medical imaging systems are almost always computer controlled and coordinate data may conveniently be obtained by means of an external connection such as a network connection. In embodiments of the system, however, information (image and/or position data) may be captured from a video or image output of the imaging system, for example by frame grabbing and, if necessary, character recognition. This simplifies interfacing and improves compatibility with some imaging systems, in particular those without a network connection. In embodiments therefore, the system includes a system to obtain image and/or coordinate data from a video feed from said imaging system.

As mentioned above, the medical signal processing system may identify changes including but not limited to ischemic changes and/or rapid changes in heart rhythm. In some embodiments of the system the medical signal processing system uses the captured image of the internal organ to help identify an abnormality. More particularly in the case of a heart the captured, say, x-ray fluoroscopy image can be used to identify the location of the catheter, for example whether it is approaching from the left or right side of the heart. Since the "crock" of the heart is on the right atrial ventricle, the abnormality identified may be a change in rhythm when the surgeon is approaching from the right but an ischemic change when the surgeon is approaching from the left. More generally the detection of the abnormality can be made more sensitive in response to the region of the imaged organ on which the medical procedure is being performed so that, say, sensitivity to a rhythm change can be increased when the surgeon is working on the right side of the heart and/or reduced when the surgeon is working on the left side of the heart. Sensitivity to an ischemic change similarly be varied in response to the region or side of the imaged organ on which the procedure is being performed. Again, more generally, in a case where the captured medical signal data comprises data associated with different regions of the imaged organ (as is typically the case with ECG data) abnormalities in a region on which the doctor is working are most likely to be caused by the doctor and therefore the sensitivity of the abnormality determination can be adjusted to increase (or decrease) the sensitivity to signals associated with the region on which the doctor is working as compared with signals from other regions of the organ.

The following describes an embodiment ofa method of displaying medical data, the method comprising: inputting, from an image capture system, captured image data representing an image of an internal organ of a patient; inputting medical signal data from said internal organ of said patient; processing said medical signal data to identify an abnormality; and displaying a captured image of said internal organ of said patient together with a graphical representation of said abnormality.

Processor control code may be provided, in particular on a carrier, for example as described above, to implement the above method.

The following describes an embodiment ofa method of determining a cardiac signal from an ECG signal, the method comprising: identifying a maximum or minimum value of a signal derived from said ECG signal within a time Window comprising a plurality of cardiac cycles; identifying peaks of said derived ECG signal within said time window by determining other parts of said signal within said time window within an amplitude range of said maximum or minimum value; and determining an average cardiac cycle signal for said time window by averaging signals from said plurality of cardiac signals time-aligned using said peaks.

Here "peaks" includes troughs - that is a peak may be positive or negative. Preferably the averaging comprises "auto cortelating". The signal derived from the ECG signal preferably comprises a filtered version of the ECG signal, for noise reduction, although (depending upon the source of the ECG signal) an input signal could be used directly.

The method may be applied repeatedly to distinguish a foetal heartbeat from that of the mother, by first capturing the combined signal, then applying the method to identify the mother's cardiac signal (which because it is larger it will be acquired first), subtracting this off, then applying the method again to determine the foetal cardiac signal. Because, even with twins, one foetal heartbeat will tend to dominate this technique may be employed where there is a plurality of foetuses and may be applied repeatedly (iteratively or recursively) to determine the cardiac signal for each foetus. Data analysis such as ST analysis may then be applied to the extracted averaged cardiac cycle signal(s).

The above described embodiments may be combined in any permutation.

Processor control code may be provided, in particular on a carrier, for example as described above, to implement the above methods.

An embodiment further provides a medical signal processing system including an ECG signal input (which may be a digital data input), a processor, and stored computer program code for coded the processor to implement the above described method.

Broadly speaking we will describe a system which facilitates automatic detection of specific pathological heart conditions such as ischemia. Embodiments of the system monitor signals coming from ECG electrodes, and these signals are analysed in real-time looking at both spatial and temporal correlations in order to detect early signs of ischemia (and/or other pathological heart conditions) for example as recommended by the guidelines of the American Heart Association and/or Canadian Heart Association.

If a pathological condition is detected and validated by the system the graphical representation of the heart will change gradually in accordance with the severity of the situation, and with a clear indication of the topographic part of the heart that is suffering. This is a dynamic process and embodiments of the system are capable of analysing, modelling, and showing on screen the evolution of a cardiac event. Preferably an acoustic alarm is triggered if the alarm level is determined to be severe enough to require immediate intervention. If a patient is at home an emergency phone call (or other alert) may be issued automatically. This facilitates early and timely diagnostics of potentially deadly conditions, and could be life saving: early diagnostics enables early access to treatment and hence increases chances of survival and recovery. Furthermore the automatic detection capability of the system combined, in embodiments, with a simple and intuitive output, facilitates non-specialist healthcare professionals monitoring a patient and reacting quickly by calling a specialist when required, thus helping to make optimum use of the generally scarce resources available in healthcare systems.

Before beginning a detailed description of a preferred embodiment of the system it is helpful to outline its operation.

Initially a set of ECG signals are acquired, for example by standard instrumentation, and then filtered, again conventionally, for noise reduction typically such filtering includes applying a narrow 50Hz or 60Hz band cut filter to attenuate grid mains pickup, applying a band pass filter over the frequencies of interest, generally 3Hz to 30Hz, and removing any *DC* offsets. Processing to determine an average cardiac cycle signal then begins.

As a first stage a time window is defined (for example by a default value such as five seconds optionally user-adjustable) either moving or successively applied to the input, and then a peak (maximum or minimum) within this window is identified. Other peaks within a range, for example up to 10 percent less, are then also identified within the window and preferably filtered by applying a timing condition- for example selecting a peak to discard where the separation of two peaks is less than a window of such as 100ms. In cases of doubt a peak can be removed to ensure that mainly good data is used for alerting. Then an auto correlation procedure is used to determine an average signal shape by using the identified peaks as reference points to, in effect, overlap the signals within the time windows. Here "peak" includes a negative peak or trough. Time synchronisation for one channel of a set of captured ECG signals can be used to time-align signals for each channel to overlap a set of cyclic cardiac signals for each channel to determine an average signal shape. This is because all the ECG signals are synchronies.

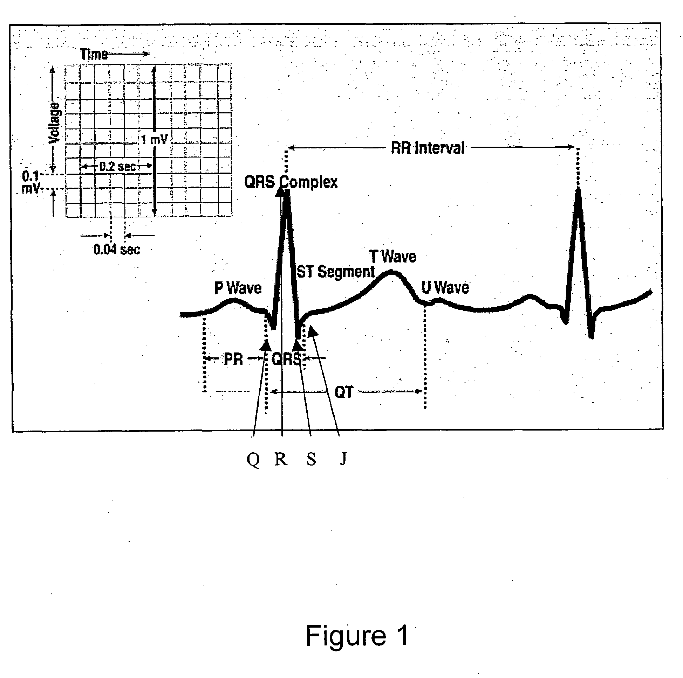

An output of the above procedure is an average cardiac signal for each channel. An example of such a signal is illustrated in figure 1: Figure 1 is a representation of an electrical signal that is generated by the patient's heart (for example for Lead 1 on a standard 12 lead ECG). Normally the signal present a number of "electrical" waves called P Wave, QRS complex, T Wave and U Wave. The QRS complex is joined to the T Wave by the so-called ST segment which starts from the J point. The elevation/depression of the J point compare to the isoelectric level can be used as indicator of an abnormal clinical condition.

For a mother and baby the average mother signal can be subtracted from the combined captured signal and then the procedure reapplied to determine the signal for the baby. This may be repeated if there are twins.

Referring to figure 1, there is a small flat region known as the isoelectric floor just before point Q; the isoelectric signal can be determined from the captured waveform by identifying the minimum Q (for example by first determining the location of maximum R) and measuring the level of the signal at a time interval such as 40ms (default, user-adjustable) before Q. Again referring to figure 1, the ST segment also has a small flat region just after S, and the level of this region can be determined by finding point S and then moving forward by a time delay, for example 30ms (default, user-adjustable). The difference between the ST level and the isoelectric floor level is known at the ST level (elevation or drop) and may be measured in terms of a voltage difference. However doctor's are used to seeing ECG traces on paper (inset in figure 1) in which case the ST level may be presented in terms of millimetres (this being acceptable because traces are often plotted on a graph paper with 1cm in the Y axis being equal to 0.5 mV and 1 cm in the X axis being 200ms, see insert on Figure 1). To determine whether or not the ST level is normal the ST level may be compared with an absolute threshold or, if the signal is small, by determining the level as a percentage change with reference to a peak signal level. The above procedure is preferably performed for each of the ECG signal channels. Alternatively other, conventional algorithms may be employed.

We next outline processing of the stream of ST data provided by each ECG signal channel to provide a graphical display.

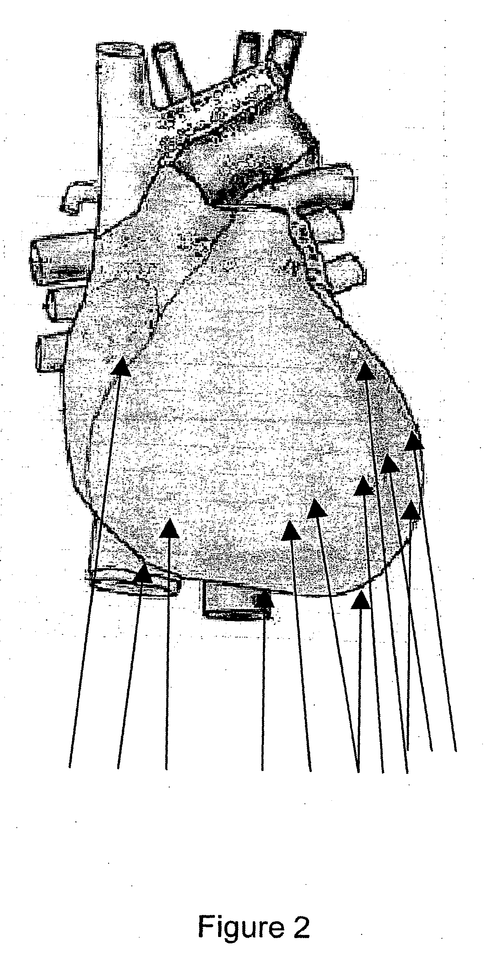

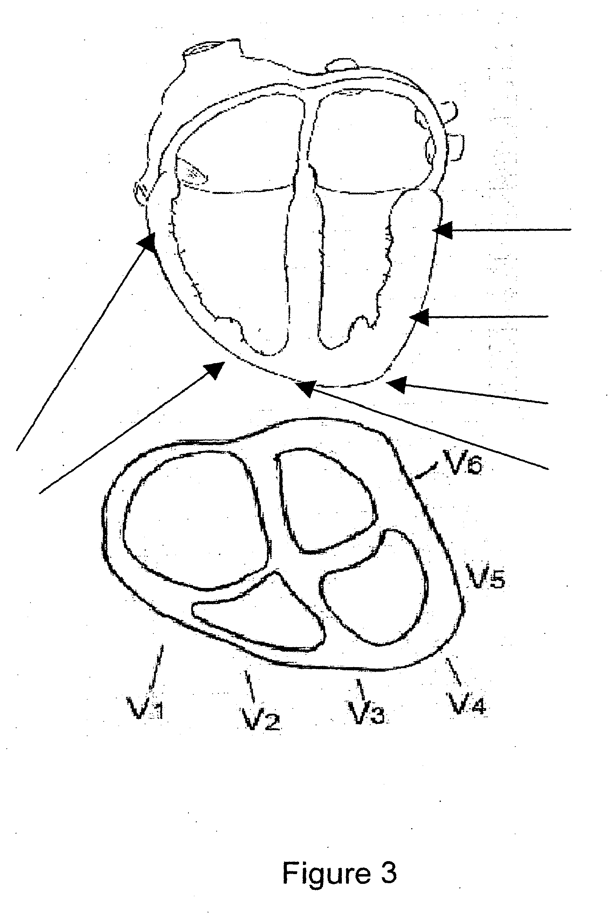

A set-up procedure maps the ECG lead positions to a graphical representation of a heart. Figures 2 and 3 show lead positions (arrows) for twelve lead ECG signal capture on a 3D heart image and on a 2D heart cross-section respectively (or more precisely, positions to which leads map). The coordinate system used is preferably that of the graphical representation of the heart and a table is provided associating each lead with a default position. Each lead is displayed as an icon (for example "V1" and so forth) which can, if desired, click-and-drag positioned by a doctor thus updating data in the table. Preferably a contour of the display part image is also defined, either automatically, semi-automatically (with user intervention) or manually, to facilitate subsequent graphical processing by defining a boundary of the image onto which data derived from the ECG signals is to be mapped.

A three-dimensional coordinate system may be employed, projected into two-dimensions for display, but preferably, for speed, the displayed image is, in effect, two-dimensional, albeit being a representation of a three-dimensional organ. The initial set-up may be performed in three-dimensions, for example to facilitate rotation of the image, and then projected into two-dimensions for providing a changing graphical display as described below.

A result of this procedure is a graphical display of the heart on-screen in two and/or three dimensions, together with a set of lead position data relative to this display, showing electrode positions on the heart walls (from outside or in cross-section). This type of display is helpful as it is clinically meaningful. Preferably a two-dimension grid is defined (invisible but overlaying the display) and the system works in this reduced resolution coordinate system, again to speed processing. Thus preferably the lead positions are located at the nearest grid positions.

Broadly the ST data for each lead is displayed at the lead position (the grid position nearest the lead position), using the grid. In other embodiments, however, the graphical image of the heart could be directly modified, for example by imposing local colour changes directly on the heart 3D representation/image.

For each ECG signal the system determines whether or not the signal is abnormal, more particularly assigning one of a plurality of alarm levels to the signal according to a plurality of pre-defined (default, user-adjustable) ST level ranges. Preferably a filter is applied to remove spurious events, by employing a validation time-window (user-adjustable between, say, ten seconds and three minutes with a default at, say, 90 seconds). In one embodiment an abnormality on a channel must continue longer than this time-window in order to be "validated" as such. In another embodiment a counter is implemented to count abnormal events (an abnormal ST level of a heart cycle signal) as, say, positive (for example +1) and normal events as negative (say -1), the problem being validated if a (preferably user adjustable) threshold is reached within a time-window.

Where an abnormal event has been validated the gravity of the event (alarm) can be determined by applying one or more tools or thresholds. For example an ST level elevation of one signal may provide an alarm if more than 1mm and a warning if between 0.5mm and 1.0mm; an ST level drop of more than 2mm may provide an alarm, and a drop of between 1mm and 2mm may provide a warning. Alternatively percentage changes may be employed to determine a gravity or severity of an "alarm" (abnormality). An identified abnormality is graphically displayed dependent upon its gravity, for example using colour (orange for warning, red for alarm) and size. For grave alarms an acoustic signal may be triggered and/or a message such as "Emergency" or "Call the Doctor" may be displayed and/or an automatic call to a doctor may be made.

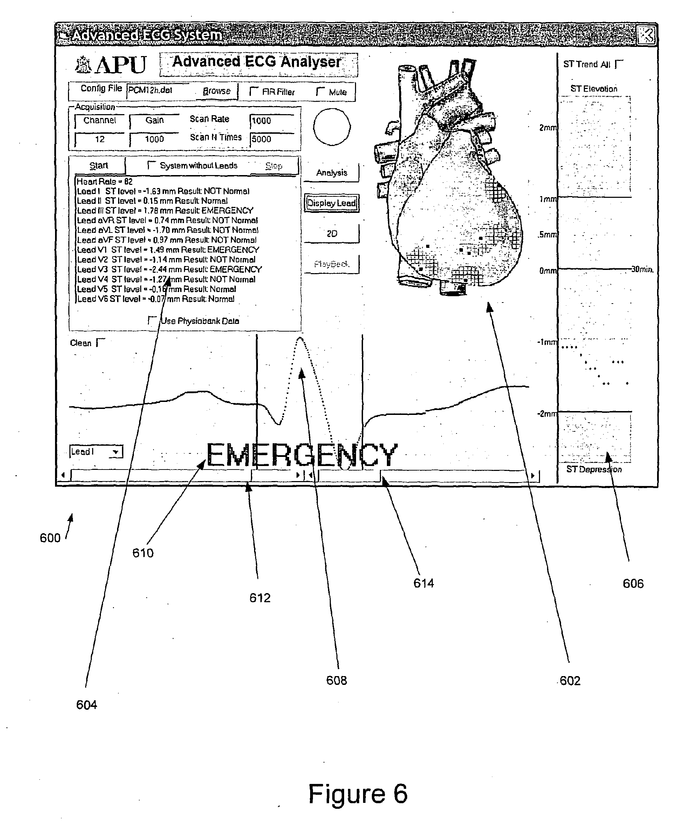

As well as a graphical display of the organ each ECG signal may be displayed on a line on which normal and abnormal ranges are marked (a bar graph type abnormality indication), and/or one or more averaged ECG signals may be displayed colour coded according to abnormality (figure 6).

To provide the graphical display of detected abnormalities of ST level of one or more of the ECG signals the above described grid is preferably employed, for each grid point determining a (weighted, moving) average of the current status/level of the point, the level of neighbouring points, the history of the point and neighbours. The 2D (or in variants 3D) grid is scanned, taking each point in turn, and determining a new level for the point. An actual electrode point is not averaged with the surrounding points (but the surrounding points do take the actual electrode point in their average). The effect of this procedure can be likened to gradually lifting a sheet of material (the grid) with a set of actuators at the lead positions - as the actuators rise the sheet or grid points around these positions actually increase in level, falling again as the actuators (ST levels at the electrode points) fall. This procedure has the consequence of producing regions of the grid which grow with persistence/level of a detected abnormality and, where two regions merge; providing a degree of mutual enhancement. In one preferred embodiment a colour for each grid point is determined based upon a (weighted moving average) calculated level of the point, for example using green for a normal (electrode) point, orange for a warning range and red for a danger range. If a level returns to a normal range another colour such as purple is preferably used instead of green to indicate the previous abnormality.

Preferably a warning indication incorporates a degree of hysteresis. Thus preferably there is a threshold set so that when the alarm level falls "low enough" a point is automatically re-set to a non-alann condition (purple colour/state) while when the alarm level rises above this minimum threshold level the point changes colour (say from green to yellow) but if the level is lower than the threshold level then its alarm level is forced back to null. This give a hysteresis to the system so that that alarm is "set" only if it has a sufficient level of severity.

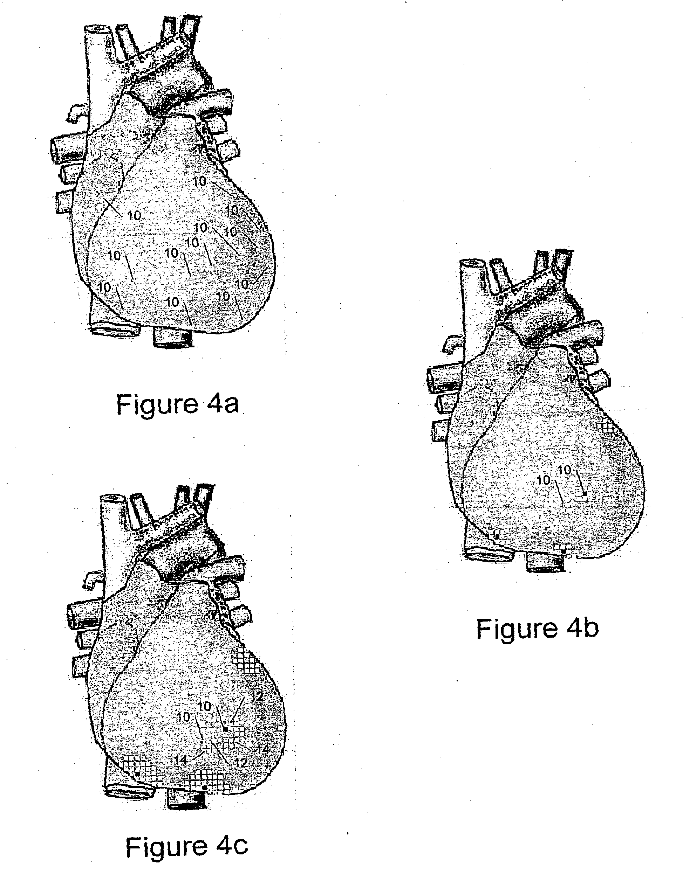

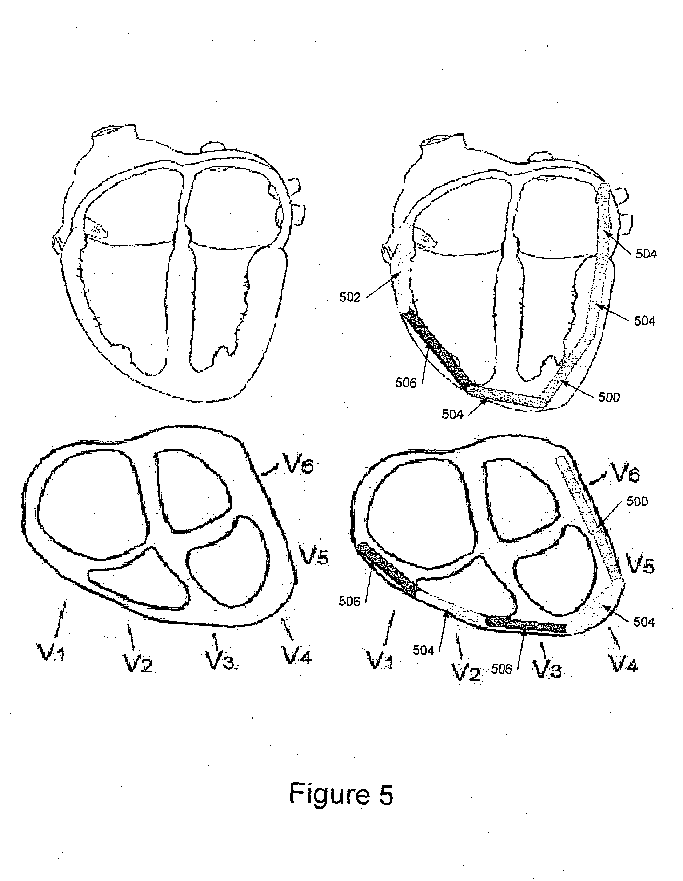

This grid data, and/or the data from which it is derived, is preferably also stored in a history database to facilitate past event analysis by playing back the recorded patterns. Figure 4 shows a time series of 3D images of a heart and a superimposed graphical display of abnormalities; figure 5 shows a time series of 2D images of a cross-sectional view through a heart with a superimposed graphical display of abnormalities. Figure 6 shows one example of a combined user interface display of a system according to an embodiment of the present invention.

As can be seen in figure 4 the time series of a 3D images of the heart showing a dynamic abnormal condition which is developed in the heart walls and can be easily interpreted by the unspecialised health professional. The analysis can be carried out in greater detail by the specialist using the available playback function and looking at the trend of the ST analysis as shown for example on the right of figure 6. Figure 5 shows a time series of a 2D cross-section of the heart.

In more detail points 10 on figure 4 indicate electrode positions which, in figure 4a are green and which progressively become orange, then red in figures 4b and 4c. As an abnormal condition continues and develops in the signal from some of the electrodes the graphical abnormality display gradually spreads out from the electrode point and, preferably at the same time progressively changes colour. Thus, for example, in figure 4c the region 12 is coloured orange surrounding central red electrode point whilst the more distant region 14 is coloured yellow (there may be a progressive change in shade of colour from the electrode point outwards. Figure 4c also illustrates a situation where respective abnormal areas from an adjacent pair of electrode points have spread to overlap, reinforcing one another in the region of overlap.

Figure 5 shows similar representations in two dimensions in which abnormalities are indicated by a line (and/or colours) extending about an electrode position according to severity and/or duration of an abnormal condition. In the example Figure, lines 500 are green, line 502 is yellow, lines 504 are orange (as illustrated, two different shades) and lines 506 are red.

In embodiments, when the abnormality ceases the tissues involved (which where graphically highlighted during the abnormality) may not return to a "normal" condition but may be left with a shaded graphical representation to highlight the fact that although at present the situation is normal this was not the case in the past. This function allows the doctor to see that something unusual has gone on and; with a historical playback facility, the doctor can go back and look in detail at what has happened.

In some preferred embodiments the system uses different colour codes to represent different heart walls conditions related to ischemia. So, for example a first set of colours, say yellow-orange-red, may be used to highlight change in the signals indicative of leading to acute myocardial infarction while a second set of colours, say sky_blue-deep_blue may be used to indicate one or more signals associated with lack of oxygen in the cardiac muscle, which indicates an onset of an ischemic event.

Figure 6 shows an example of a screen display 600 which includes a graphical abnormality display 602 (in this example a 3D view as shown in figure 4 with, preferably a 2D view shown in figure 5 as an option). Display 600 also indicates results 604 of a severity analysis of incoming data and provides a bar graph type display 606 providing a trend analysis. An averaged captured signal 608 is also displayed. A text alert warning message 610 may also be provided. Preferably controls, in this embodiment sliders 612, 614, are provided for setting the time position of the isoelectric (base line point and of the "J" point, see figure 1).

We will now describe in more detail an embodiment of apparatus and flow diagrams for software to implement the above-described system.

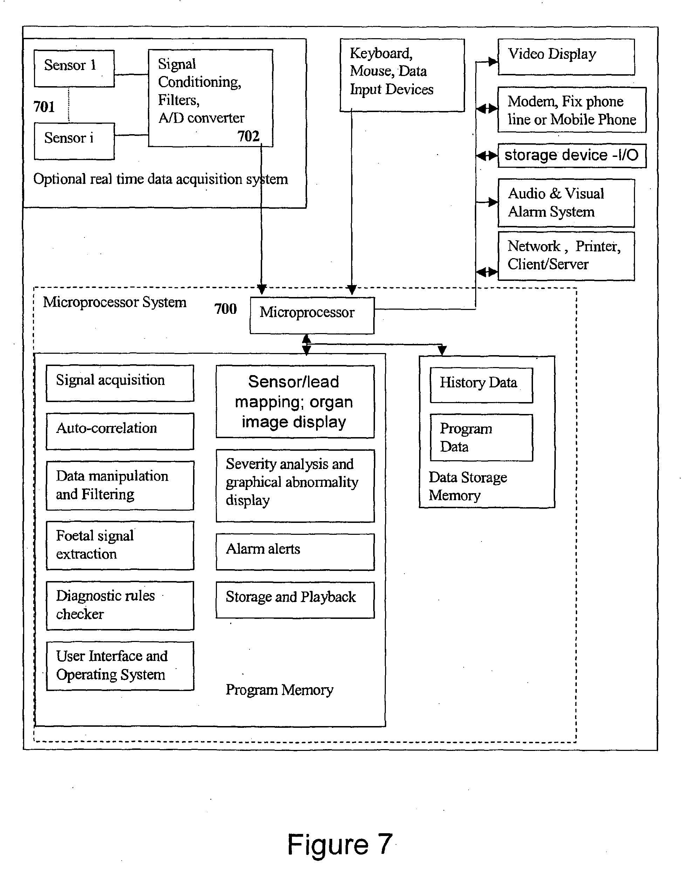

Figure 7 shows a block diagram of an embodiment of a system according to the present invention.

Referring to Figure 7, a microprocessor system 700 comprises program memory storing program components for signal acquisition; auto-correlation; data manipulation and filtering; foetal signal extraction; diagnostic rules checker; sensor/lead mapping (mapping sensor/lead positions to heart/organ 3D positions, including organ image display and grid definition); severity analysis and graphical abnormality display; alarm alerts; storage and playback; and user interface and operating system. The program memory is coupled to a microprocessor; data storage memory is also coupled to the microprocessor for storing history data and program related (working) data. The microprocessor is coupled to a bus which is preferably coupled to one or more of a video display, a modem (fixed phone line or mobile phone), a storage device and/or input/output for a storage device, an audio and/or visual alarm system, a network connection, a printer, and optionally other client/server connections. The microprocessor is also coupled to one or more user input devices such as a keyboard, mouse or other data input devices. Optionally an embodiment of the system includes a real time data acquisition system shown in figure 7 coupled to the microprocessor system 700. This real time data acquisition system may include inputs for one or more sensors and associated signal conditioning, filtering, and analogue-to-digital conversion modules.

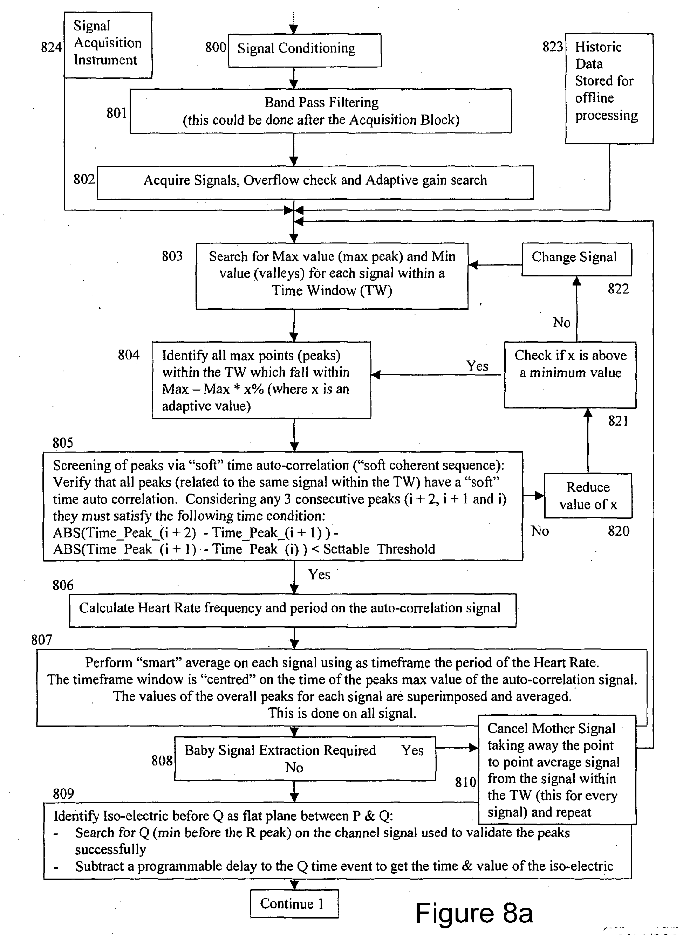

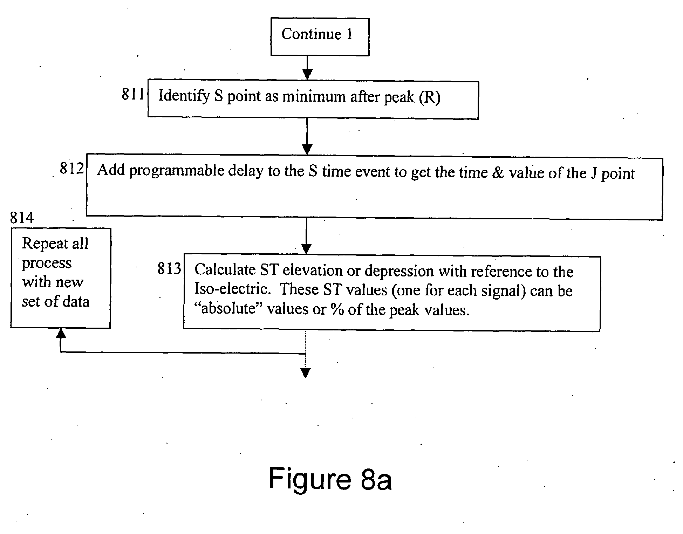

Figure 7 shows that the system may have a number of input sensors (701) or leads which are connected to a signal conditioning, amplification and filtering block (702). The signals are processed by an analogue to digital converter and are read by the microprocessor. Alternatively the microprocessor may read this data from an external storage device or internal data storage memory or from a generic I/O port connected to an external instrument or an other computer. The microprocessor system (700) processes and analyses the data following the flow diagrams in Figures 8, 9 and 10.

Figure 8 shows a flow diagram of a signal conditioning and analysis procedure for the system of figure 7.

Referring to figure 8, the signals read by the microprocessor (700) can come from a variety of sources:

(a) from an optional real time data acquisition system made up by a Signal conditioning block (800), a filter (801) to perform band pass filtering (this could be done after the acquisition block) and an Analogue to Digital converter (802) preferably with programmable gain which is set in order to avoid overflow while maximising the signal amplitude by an adaptive function.

(b) from a previously stored data file containing the patient data (823) - historic data stored for offline processing.

(c) from a signal acquisition instrument (824).

The user has the option to set a Time Window (TW) (803) or use a default value of say 5 seconds [search for Max value (max peak and Min value (valleys) for each signal within a Time Window (TW)]. The TW can be fixed (for example for a 5 second window the TW is set from 1 second to 5 second then from 6 seconds to 10 seconds and so on) or the TW can be a rolling window with a user settable increment (say 1 second). In this later case a 5 second TW would select 1 second to 5 second and then 2 second to 6 second and so on. These options allow the system to be more versatile and able to analyse different types of signals.

Once the signals are read for the defined TW a search for the maximum value (MAX) and minimum value (MIN) is carried out (803). Then a search (804) is carried out in order to identify all the QRS complexes, still within the TW. The selection criteria is that the value of each peak (or valley) should fall within an adaptive window between MAX and MAX*x% (or MIN and MIN*y%) where x is a number starting at 0.95 and moving down to a final threshold of say 0.6 (while y is: $1.1 < y, < 1, 4$), [Identify all max points (peaks) within the TW which fall within Max - Max * x% (where x is an adaptive value)].

Once all the peaks (and valleys) have been identified a "soft" time autocorrelation algorithm (805) verifies that indeed the QRS complexes have the correct time relationship so as to discharge any noises that are not correlated with the heart beat. This is done by looking at the time dependency which must exist between successive QRS complexes. [Screening of peaks via "soft" time auto-correlation ("soft coherent sequence"; Verify that all peaks (related to the same signal within the TW) have a "soft" time auto correlation. Considering any 3 consecutive peaks (i+2, i+1 and i) they must satisfy the following time condition:

$$ABS(Time+Peak\_(i+2) - Time\_Peak\_(i+1))-$$

$$ABS(Time\ Peak\ (i+1) - Time\ Peak\ (i)) < Settable\ Threshold\}$$

If the peaks (or valleys) fail to correlate the value of x (and y) is changed (820) (reduced). Then the process (804, 805) is repeated. If the value of x (or y) gets to its threshold (821) [Check if x is above a minimum value] then the signal is deemed to be too noisy (not above minimum value) and another signal (if available) is therefore selected (822) (change signal) and the process restarts from (803).

The outcome of the above process is that if one signal/lead on all valid QRS complex within the TW have been identified and therefore the heart rate frequency can be calculated (806) [Calculate Heart Rate frequency and period on the auto-correlation signal]. At this point an autocorrelation procedure (807) is used to perform an average on each signal using as timeframe the period of the heart rate [Perform "smart" average on each signal using as timeframe the period of the Heart Rate. The timeframe window is "centred" on the time of the peaks max value of the auto-correlation signal. The values of the overall peaks for each signal are superimposed and averaged. This is done on all signals]. The timeframe window is centred on the time of the peak (R peak in the QRS complex) so that all peaks (and valleys) related to the same signal are overlapped and averaged over the entire timeframe window. Given that all signals coming from the heart on different leads have synchronous information (because the source of all the signals is the same in the case of the heart) then the time synchronisation information which has been calculated on one signal can be used to time-align signals for each channel overlapping a set of cyclic cardiac signals (for each channel) and determine an average signal AV S (for each channel) across the timeframe window.

If the organ under analysis is not the heart then each channel can be considered independently and time-aligned using only information about itself.

In the case of a pregnant woman if the user is interested in analysing the signal coming from the baby (808) then the averaged signal for each channel can be taken away along the entire TW thus cancelling the mother signal (810) and the entire process can be reapplied to extract the signal for the baby. [Cancel Mother signal taking away the point to point average signal from the signal within the TW (this for every signal) and repeat.]

This process works effectively because the signal of the mother is not synchronous with the one from the baby and the signal of the mother is also much stronger therefore the system will in the first instance lock to the mother's signal. Once this gets cancelled (810) the process is repeated (recursively) until the signal of the baby is extracted. If there are twins (or triplets and so on) the process is repeated again leveraging the fact that the heart's signals from different babies will not be synchronous to each other and will have different amplitude due to the different position they are relative to the position of the sensors.

The averaged signal (AVS) of a user defined channel/lead (with default being the lead on which the QRS search has been successful) is analysed (809) in order to identify the time of the isoelectric level. [Identify Iso-electric before Q as flat plane between P & Q: - Search for Q (min before the R peak) on the channel signal used to validate the peaks successfully, - Subtract a programmable delay to the Q time event to get the time & value of the iso-electric] The isoelectric level is normally the level of a small flat plane between the P Wave and the Q Wave (see figure 1) while the time is just the relative time between the isoelectric and the peak of the QRS complex. A search of the Q Wave is carried out (as the minimum before the R peak) on the channel used to validate the peaks, and a programmable (user defined) delay to the Q time event is subtracted so as to identify the time of the isoelectric. The user can overrun this setting via a graphical interface (figure 6) forcing the item of the isoelectric to be set at any point of the averaged signal TW.

Next to the ST segment (figure 1) is identified and analysed in order to identify the "J" point, the point at which the ST line has a knee and start to "slowly" rise towards the T wave in a normal cardiac cycle. The "J" point search algorithm is similar to the isoelectric algorithm. Looking at figure 8b flow diagram the S point is identified (811) via a minimum (or maximum) search [Identify S point as minimum after peak (R)] and then a programmable (user defined) delay is added (812) so as to identify the time of the J point [Add programmable delay to the S time event to get the time & value of the J point]. Alternatively a slope algorithm can be used, looking for when the value of the angular coefficient of the tangent to the averaged signal reaches a predefined value.

The user can overrun this J point time setting via a graphical interface (figure 6) forcing the time of the J point to be set at any point TW.

Finally the ST elevation or depression (813) is calculated for each signal/lead as the value of the J point in relation to the isoelectric value. This can be an absolute value measured in MV (or millimetre) or a relative percentage value of the QRS peak, of the peak is small for example. The user can choose which setting (absolute or percent) to use or the system can work this out automatically based on the QRS value compare to a pre-determined threshold. The process may then loop (814) and repeat with a new set of data.

Once the ST values are evaluated using the above process (figure 8) or via alternative other conventional algorithms the graphical analysis procedure is called (900).

If all the above process is unsuccessful in identifying a synchronisation sequence of peaks and therefore cannot be measure the ST values then the same process is repeated this time looking at identifying a synchronisation sequence of minimum values (i.e., the valleys or "negative peaks") with a "soft coherent" time sequence search.

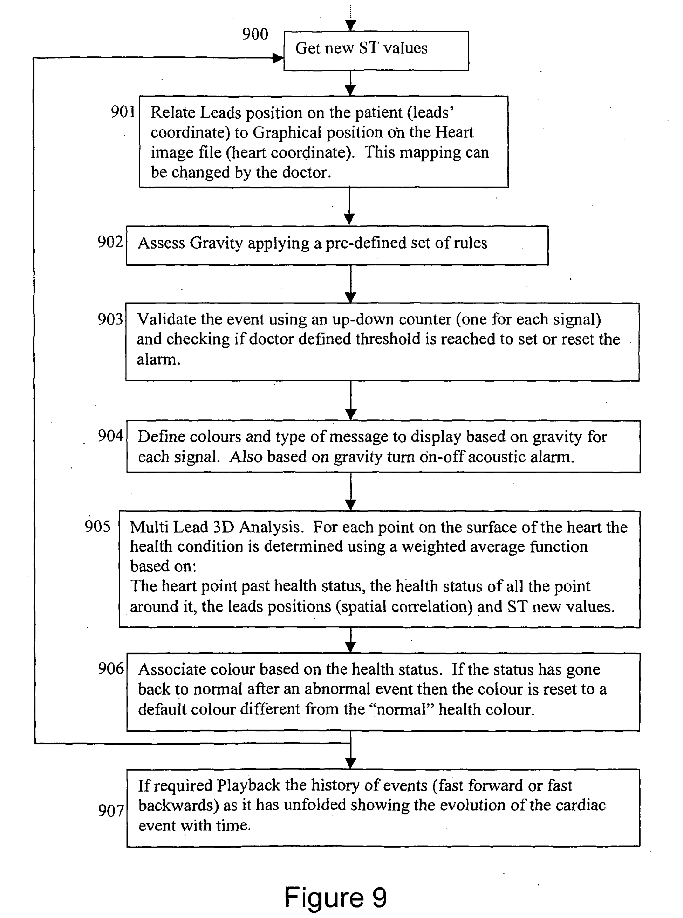

Figure 9 shows a flow diagram of a graphical display procedure for the system of figure 7.

The procedure gets a set of ST values (900) and The first task carried (901) out by the procedure is then to relate the lead positions on the patient (lead coordinates) to the corresponding heart wall position on a 3D view of the heart organ and 2D cross-section views. The system has some default setting for the most standard configurations of the leads related to the heart (or to the brain if EEG leads are used) but the doctor can modify these default positions, for example by graphically dragging and dropping the leads into the new positions.

The gravity of the condition is assessed (902) using some predefined rules or via an identification phase based on past clinical results or on fuzzy logic or neural network algorithm or a combination of them. A numerical value representing the gravity of the condition is assigned for each of the signals.

An example of a set of rules that can be applied in the case of an Ischemia algorithm detection for the heart is to check the value of the ST level (for absolute measurements (813)). If this value is between 0.5mm elevation and 1mm depression then gravity level is assumed to be "nil" or "normal". When the ST level falls between 0.5mm and 1mm elevation or between 1mm and 2mm depression (or drop) then the system will detect an abnormal condition and assume a linear relationship between the gravity value and the ST level between the 2 limits for elevation or depression. For absolute values of ST level, the Gravity attribute and value can be set following these rules:

Normal

    0.5 mm elevation > ST level < 1 mm drop; Gravity = "normal" or "nil", value = "nil"

Abnormal

    1 mm elevation > ST level >= 0.5mm elevation; Gravity = "abnormal-elev", 0 < value <= 255
    2 mm drop > ST level >= 1mm drop; Gravity = "abnormal-drop", 0 < value <= 255

The value of the gravity can be assigned using a liner interpolation function (or a more sophisticated function if required).

Emergency

    ST level >= 1 mm elevation; Gravity = "Emergency-elev", value = 0
    ST level >= 2 mm drop; Gravity = "Emergency-drop", value = 0

The gravity is validated (903) using for example an up-down counter (one for each signal) and checking if the user defined threshold is reached before setting (or resetting) an alarm condition. In this way short transients due for example to the patient moving, are discharged and the alarm is raised only if a persisted condition occurs.

Clearly there are many different algorithms that can be used to filter out the unwanted transients; the one described is just an example.

Based on the gravity a colour is assigned (904) to each point of the heart directly related to the lead, from green (normal condition) to yellow-orange (abnormal condition) to red (critical condition). If an RGB display is used (base colours red, green, blue) with say 255 values for each base colour then the normal condition would be "0,255,0" while the critical condition would be "255,0,0" and the abnormal condition would be "255,0,xxx" where xxx is a number between 0 and 255. The value of xxx will be dictated by the gravity, if the gravity is "near normal" then xxx will have a value near 255 and the colour will be yellow while if the gravity is "near emergency" then xxx will have a value near 0 (orange colour) therefore an algorithm can be used to assign the value of the RGB for each gravity value.

Also based on the above analysis (gravity) an acoustic message/alarm can be raised and/or one or more messages displayed and (remote) assistance can be called in.

A multi-lead 3D analysis is carried out (905) on all the points of the heart (except those directly related to the leads) in order to correlate the information coming from the gravity analysis on the leas with the anatomical and topological information on where the leads are (spatial correlation) and the evolving nature of the heart (or brain) health status with time (temporal correlation). Each point of the heart walls is taken in turn and a weighted average function is applied in order to determine its current health status taking into consideration the past health status, the health status of (preferably all) the points next to the leads positions and the gravity analysis at the lead's position. [For each point on the surface of the heart the health condition is determined using a weighted average function based on: The heart point past health status, the health status of all the point around it, the leads positions (spatial correlation) and ST new values.]

The gravity value of the point being examined is assigned by performing a weighted average of the gravity of all the points (in the heart walls) around the point being considered. A (user defined) threshold is set so that only if the gravity value exceeds this threshold then the gravity value is considered valid and the new value gets assigned otherwise the gravity value gets reset to "nil" if the previous value was "nil" or to a "purple" condition if the previous value was an "abnormal" or "emergency" value (i.e. above the threshold).

The value of the threshold is not fixed but depends upon the distance of the given point from each lead. The closer the point is to a lead the lower is the threshold (making the point more reactive to the changes coming from the lead) while the further away the point is from the nearest lead the higher the threshold is making the point less sensitive to the lead(s).

Based on this analysis a health/gravity value is given to the point (906) and using the same rules as described before (904) a colour is associated to the particular point of the heart. [Associate colour based on the health status. If the status has gone back to normal after an abnormal event then the colour is reset to a default colour different from the "normal" health colour.] The threshold operates to ensure that if the gravity does not go above a particular value then the new value is reset to normal ("nil"). If the status was previously "abnormal" or "emergency" and it goes back to "normal" and the colour of the point gets set to a predefined (and user defined) one, say "purple" for example, but different from green (or transparent) to indicate that although the status is "normal" at present, this region of the heart has suffered an abnormal event.

Preferably all data is stored on files for analysis at a later stage of batch analysis. The user can then (907) playback the history events at a user defined time acceleration rate (default 1 minute = 1 second) and the system allows the user to fast-forward, stop and fast backwards (similar to a common VCR/DVD function) in order to see how events have unfolded showing the evolution of the cardiac event with time.

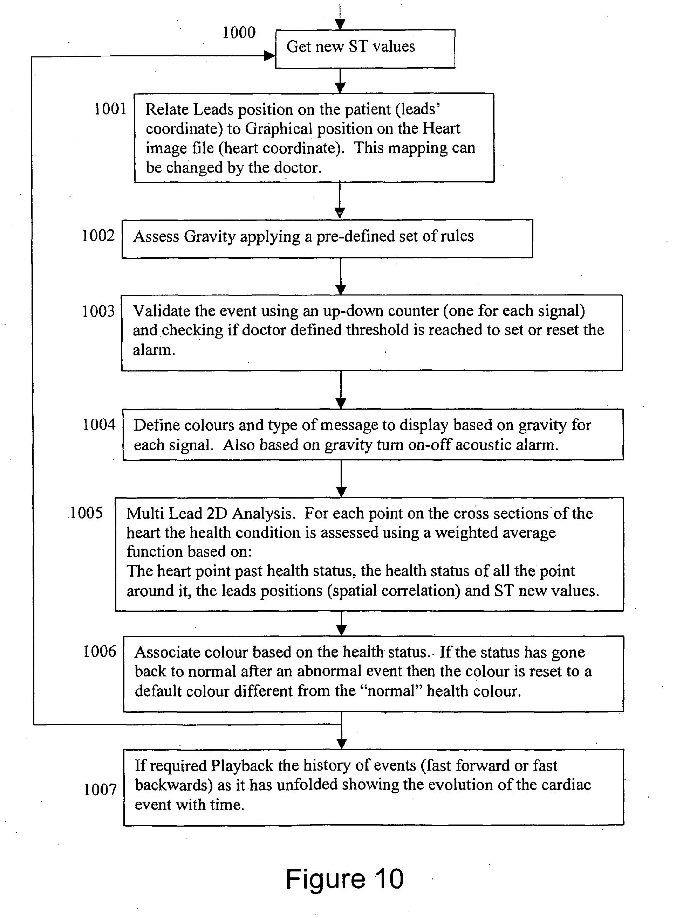

Figure 10 shows a flow diagram of a 2D graphical abnormality display procedure to display a 2D cross-sections of the organ under test (heart or brain for example). It should be understood that the algorithms shown in figures 8, 9 and 10 run in parallel so that the 3D views and the 2D views are available to the user at all times. As it can be seen there is very little difference between the flow diagram on figure 9 and figure 10 except for the block (905) and (1005) [For each point on the cross sections of the heart the health condition is assessed using a weighted average function based on: The heart point past health status, the health status of all the point around it, the leads positions (spatial correlation) and ST new values.].

A multi-lead 3D analysis is carried out on each heart wall visible on the cross-sections 2D views. Typically for the heart the doctors look at the frontal cross-section and a pre-cordial cross-section (see figure 3 and figure 5, for example) so these are the default 2D views.

Based on the gravity a colour is assigned (1004) to each segment of the heart directly related to the leads, from green or transparent (normal condition) to yellow-orange (abnormal condition) to red (critical condition). If an RGB display is used (base colours red, green, blue) with say 255 values for each base colour then the normal condition would be "0,255,0" while the critical condition would be "255,0,0" and the abnormal condition would be "255,0,xxx" where xxx is a number between 0 and 255. The value of xxx will be directed by the gravity, if the gravity is "near normal" then xxx will have a value near 255 and the colour will be yellow while if the gravity is "near emergency" then xxx will have a value near 0 (orange colour) therefore an algorithm can be used to assign the value of the RGB for each gravity value. Also based on the above analysis an acoustic message/alarm can be raised and (remote) assistance can be called in.

A multi-lead 2D analysis is optionally carried out (1005) on all the points to the heart on the cross-sections (except those directly related to the leads) in order to correlate the information coming from the gravity analysis on the leads with the anatomical and topological information on where the leads are (spatial correlation) and the evolving nature of the heart health status with time (temporal correlation). Each point of the heart walls is taken in turn and a weighted average function is applied in order to determine its current health status taking into consideration the past health status, the health status of all the points next to it, the leads position and the gravity analysis at the leads positions. Based upon this analysis a health/gravity value is given to the point (1006) using the same rules as described before (1004) and the new colour is associated to the particular point or segment of the heart. A threshold is applied so that if the gravity does not go above a certain value the value is reset to normal. If the status was previously "not normal" and it goes back to "normal" the colour of the point gets set to a predefined (and user defined) one (different from green or transparent) to indicate that although the status is "normal" at present, this region of the heart has suffered an abnormal event.

Referring again to Figure 6, this shows an example implementation of the system, where the extracted and averaged over TW signal (user selectable) is shown on the bottom window, and where the user can set the time of the isoelectric level and the "J" point. Also a user defined warning/alarm message can be issued and the trend of the gravity analysis is shown as a function of time for a user selectable lead (or for all leads). The 3D view (and/or a 2D cross-section view) is present in the centre while a text window with detailed information about the severity/gravity analysis is also available.

Once a patient is diagnosed with Ischemic Heat Disease, he/she may require angiography to gain a detailed, clear and accurate picture of the cardiac blood flow in the coronary vessels. If the diagnostic procedure finds areas with sever arterial narrowing, then it is usually followed by angioplasty, a surgical procedure to re-open the arterial vessels. Both these invasive procedures typically make use of x-ray fluoroscopy, in which generally the patient lies on a table and the x-ray machine moves around in space and captures video images of the thorax/heart taken from different angles. The images are displayed on a screen. ECG signals may be displayed on a second screen to monitor any ischemic event induced by the procedure. However the combination, preferably on a single screen or monitor, of x-ray fluoroscopy video images together with computer generated heart images as described above improves dramatically doctors' awareness of the status of the patient and therefore reduce the risks associated with these procedures. Preferably the image shows graphically the heart walls with highlighted areas that are suffering from ischemia as they evolve in real time. The graphical and captured images can be presented side by side or after an image registration stage in which the two sets of images are merged together so as to provide a unified view on one screen, displaying in real time anatomical details of the heart, including the distribution of coronary vessels, while showing concurrently any areas suffering from ischemic changes due to low blood flow.

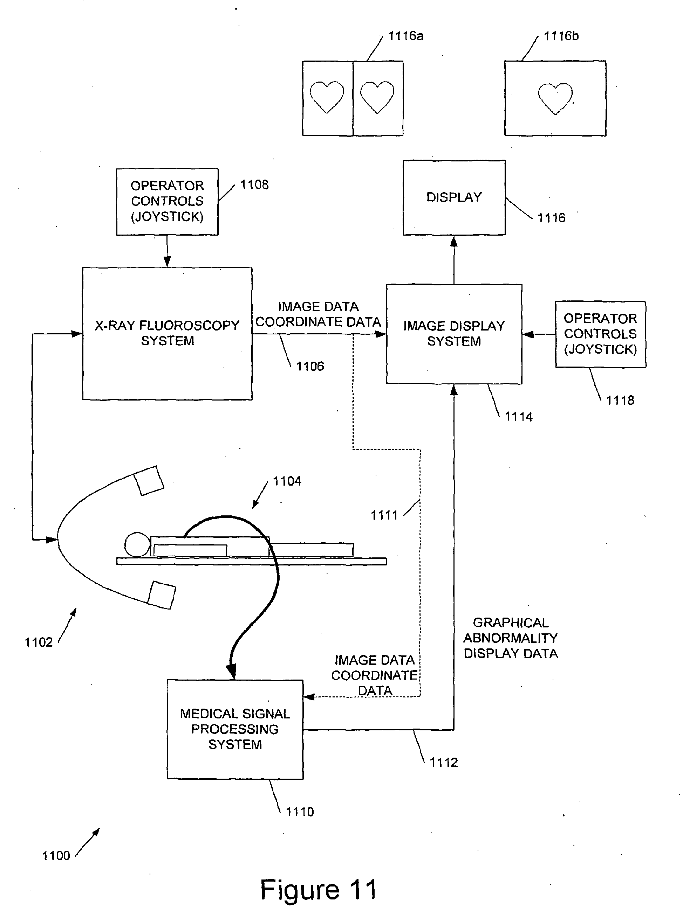

Referring now to Figure 11, this shows a block diagram of a medical image display system 1100 for displaying a graphical indication of an abnormality together with a captured x-ray fluoroscopy image, as described above.

In Figure 11 an x-ray fluoroscopy system 1102 captures images from a patient 1104 and provides an image data output, for example on a computer network connection 1106. Alternatively an image or video output from system 1102 may be employed, for example using a frame grabber (not shown) to obtain this data. The x-ray images are captured from a controllable view point selected by means of operator controls 1108, typically including a joystick. The coordinates of a captured image are also provided on network connection 1106 (or may also be captured from video data).

A medical signal processing system 1110, for example as described above with reference to Figure 7, captures ECG data from the patient 1104 and provides data for a graphical abnormality display on a second computer network connection 1112. This may comprises, for example, colour data for grid positions of a grid mapping to regions of the heart, as described above. Conveniently the output may comprise data in a similar or the same format as that stored in the above mentioned history database.

An image display system 1114 receives the captured image data on connection 1106 and the processed medical signal data on connection 1112 and provides a graphical image display output on monitor 1116. This may either comprise a split screen display 1116a or an overlapping screen display 1116b. In the latter case operator controls 1118, for example a second joystick, associated with image display system 1114 may be employed to scale and/or rotate and/or translate one of the images, typically the graphical representation of the abnormality, so that, by eye, it fits over the captured image of the heart. The image coordinates transmitted from x-ray fluorescence system 1102 may then be employed to track changes in the captured image and corresponding change the graphical representation of the abnormality, thus providing a straightforward implementation of image "fusion". Alternatively the image fusion may be performed automatically, using image XYZ, attitude and azimuth data from system 1102.

Optionally the medical signal processing system 1110 may also have a link 1111 to the x-ray imaging system 1102. In this way the medical processing system can perform simple image processing on the captured image data, for example to identify whether the catheter is on the left or right hand side of the image (the catheter stands out clearly in the x-ray image). The detection of an abnormality can then be made preferentially sensitive to, say, the general location of the catheter, since this is where detected abnormalities are most likely to be caused by the operation.

No doubt many other effective alternatives will occur to the skilled person. For example the above described techniques, although particularly useful for heart monitoring, may also be applied to other organs such as the brain. Similarly, although it is generally convenient for preferred embodiments of the method to employ electrical signals, magnetic or thermal signals may also be processed in a similar way. It will be understood that the invention is not limited to the described embodiments and encompasses modifications apparent to those skilled in the art lying within the scope of the claims appended hereto.

**Claims**

1. A medical signal processing system (700) for processing medical signal data obtained from a human (1104) or animal heart, and displaying a graphical representation of the processed data for providing an ischemia onset alert, the system (700) comprising:

a medical signal data input (701) for inputting one or more ECG signals;
a graphical display (1116); and
a signal processor (1110) coupled to said data input (701) and to said display (1116),
wherein said signal processor (1110) is configured to:

input medical signal data comprising said one or more ECG signals associated with a spatial position in or on an organ, wherein said organ is said heart;
perform at least one measurement relating to said organ using said medical signal data to provide ST level measurement data, a said ST level corresponding to a difference in voltage between two parts of a said ECG signal;
determine from said measurement data whether said measurement is normal or abnormal;
define a grid for overlaying on a graphical representation of said heart showing a region of a wall of said heart, said grid comprising a plurality of grid positions;
display, at a position on said graphical representation of said heart mapped from said medical signal data input of said ECG signal, a graphical indication (502, 504, ,506) of an abnormality at said spatial position on a wall of said heart with which said signal data is associated responsive to a said abnormality determination; and
vary said graphical representation of said region of said heart wall by changing the number of said grid positions which are occupied on the basis of a said abnormality determination.

2. A medical signal processing system (700) as claimed in claim 1 wherein said abnormality determination determines into which of a plurality of categories of abnormality or ranges of severity of abnormality said measurement falls; and wherein said graphical indication (502, 504, ,506) of said abnormality is responsive to said range or category determination.

3. A medical signal processing system (700) as claimed in claim 1 or 2 wherein said graphical representation is responsive to a history of said abnormality determination.

4. A medical signal processing system (700) as claimed in claim 3 wherein said display (1116) further indicates a past abnormality when said determination is normal and was previously abnormal and/or wherein said graphical representation indicates a duration of a said abnormality.

5. A medical signal processing system (700) as claimed in any preceding claim wherein said signal data comprises a plurality of signals associated with a plurality of said spatial positions in or on said organ, wherein said measurement and determination are performed for each of said plurality of signals, and wherein said display (1116) graphically indicates, for each of said plurality of signals, an abnormality, responsive to said measurement and determination, at a said spatial position associated with the signal.

6. A medical signal processing system (700) as claimed in claim 5 wherein said graphical indication (502, 504, ,506) for a said signal is responsive to an abnormality determination for one or more others of said signals.

7. A medical signal processing system (700) as claimed in any preceding claim wherein said graphical indication (502, 504, ,506) comprises a representation of a said abnormality which grows in size with duration and/or severity of said abnormality.

8. A medical signal processing system (700) as claimed in any preceding claim wherein said signal processor (1110) is further configured to determine, for each of a plurality of grid positions on said graphical representation of said organ, a said graphical indication (502, 504, ,506) of abnormality responsive to said determination; and preferably wherein said graphical indication (502, 504, ,506) at a said grid position is responsive to a said graphical indication (502, 504, ,506) at one or more neighbouring positions.

9. A medical signal processing system (700) as claimed in any preceding claim wherein the graphical representation comprises a shape (502, 504, ,506) which grows in extent as the abnormality determination persists or worsens; and preferably wherein said shape (502, 504, ,506) contracts in area once the abnormality determination ceases; preferably wherein when a said shape contracts in area a graphical representation of a past abnormality is left behind.

10. A medical signal processing system (700) as claimed in any one of claims 1 to 9 wherein said graphical representation of said heart comprises one or more 2D cross-sectional views of said heart, and wherein said graphical indication (502, 504, ,506) of an abnormality comprises a line on a cross-section of said heart wall; or wherein said graphical representation of said heart comprises one or more 3D views of said heart, and wherein said graphical indication (502, 504, ,506) of an abnormality comprises a highlighted region on said heart wall.

11. A medical signal processing system (700) as claimed in any one of claims 1 to 10 wherein said signal processor (1110) is further configured to pre-process said medical signal data prior to performing said measurement by identifying a maximum or minimum value of a signal derived from said ECG signal within a time window comprising a plurality of cardiac cycles;
identifying peaks of said derived ECG signal within said time window by determining other parts of said signal within said time window within an amplitude range of said maximum or minimum value;
and determining an average cardiac cycle signal for said time window by averaging in particular auto-correlating signals from said plurality of cardiac signals time-aligned using said peaks.

12. A medical signal processing system (700) as claimed in claim 1 wherein said processor is configured to determine a persisted condition on the basis of said one or more ECG signals and to perform a said change responsive to said persisted condition determination.

13. A medical image display system comprising the medical signal processing system (700) of claim 1, the medical image display system comprising:

an image capture system (1106, 1114) to capture an image of an internal organ of a patient;
a medical signal processing system (1110) to capture medical signal data from said imaged organ of said patient (1104) and to process said captured medical signal data to identify an abnormality; and
an image display system (1116) coupled to said image capture system (1106, 1114) and to said medical image processing system (1110), to display an image of said internal organ together with a graphical representation of said abnormality.

14. A medical image display system as claimed in claim 13 wherein said internal organ comprises a human (1104) or animal heart, and wherein said captured medical signal data comprises ECG data, in particular wherein said abnormality comprises one or both of a change in rhythm of said heart and an ischemic abnormality of said heart.

15. A medical image display system as claimed in claim 13 or 14 wherein said image capture system comprises an x-ray fluoroscopy imaging system, and/or wherein said image display system (1116) is configured to display said graphical representation or on said image of said abnormality on a graphical representation of said internal organ.

16. A medical image display system as claimed in any one of claims 13 to 15 wherein said image capture system (1106, 1114) includes a system to provide coordinates defining a captured view of said internal organ, and wherein said image display system (1116) is responsive to said coordinates to adjust said graphical representation of said abnormality to correspond with said view.

17. A medical image display system as claimed in any one of claims 13 to 16 wherein said medical signal processing system (1110) is coupled to said image capture system (1106, 1114), and configured to process said captured medical signal data responsive to image data for said captured image.

18. A medical image display system as claimed in claim 17 configured to identify a region of said imaged organ on which a medical procedure is being performed, and wherein said medical signal processing system is configured to perform said abnormality identification responsive to said identification of said region.

19. A medical image display system as claimed in any one of claims 13 to 18 wherein said medical signal processing system (1110) comprises a signal processor configured to:

input medical signal data associated with a spatial position in or on said organ;
perform at least one measurement relating to said organ using said medical signal data to provide measurement data;
determine from said measurement data whether said measurement is normal or abnormal; and
output data for displaying, on a graphical representation of said organ, a graphical indication (502, 504, ,506) of an abnormality at said spatial position with which said signal data is associated responsive to an abnormal to said determination; in particular wherein the graphical representation comprises a shape which grows in extent as the abnormality determination persists or worsens.

20. A method of displaying medical data to indicate ischemia onset of a heart, the method comprising:

inputting, from an image capture system (1106, 1114), captured image data representing an image of an internal organ of a patient;

inputting medical signal data from said internal organ of said patient, said medical signal data comprising said one or more ECG signals associated with a spatial position in or on said heart;

processing said medical signal data to determine an ST level measurement to identify an abnormality, a said ST level corresponding to a difference in voltage between two parts of a ECG signal;

defining a grid for overlaying on a graphical representation of a heart showing a region of a wall of said heart, said grid comprising a plurality of grid positions;

displaying a captured image of said internal organ of said patient together with a graphical representation of said abnormality at a position on said graphical representation of said heart mapped from said spatial position;

varying said graphical representation of said region of said heart wall by changing the number of said grid positions which are occupied by said graphical abnormality indication on the basis of a said abnormality determination.

**Patentansprüche**

1. Medizinisches Signalverarbeitungssystem (700) zum Verarbeiten von medizinischen Signaldaten, die aus einem menschlichen (1104) oder tierischen Herz erfasst werden, und zum Darstellen einer grafischen Repräsentation der verarbeiteten Daten zum Bereitstellen eines Ischämiebeginn-Alarms, wobei das System (700) Folgendes umfasst:

einen Eingang (701) für medizinische Signaldaten zum Eingeben von einem oder mehreren EKG-Signalen;
einen Grafikbildschirm (1116); und
einen Signalprozessor (1110), der an den Dateneingang (701) und an den Bildschirm (1116) gekoppelt ist, worin der Signalprozessor (1110) konfiguriert ist zum:

Eingeben von medizinischen Signaldaten, die das eine oder die mehreren EKG-Signale umfassen, die mit einer räumlichen Position in oder an einem Organ assoziiert sind, worin das Organ das Herz ist;
Ausführen von mindestens einer das Organ betreffenden Messung unter Verwendung der medizinischen Signaldaten, um ST-Niveau-Messdaten bereitzustellen, wobei ein solches ST-Niveau einer Differenz in der Spannung zwischen zwei Teilen eines solchen EKG-Signals entspricht;
aus den Messdaten bestimmen, ob die Messung normal oder abnormal ist;
Definieren eines Rasters zum Auflegen auf eine grafische Repräsentation des Herzens, die einen Bereich einer Wand des Herzens zeigt, wobei der Raster eine Vielzahl von Rasterpositionen umfasst;
an einer Position auf der grafischen Repräsentation des Herzens, die aus der medizinischen Signaldateneingabe des EKG-Signals abgebildet ist, eine grafische Anzeige (502, 504, 506) einer Abnormalität an der räumlichen Position an einer Wand des Herzens darstellen, womit die Signaldaten reaktionsfähig auf eine solche Abnormalitätsbestimmung assoziiert sind; und
Variieren der grafischen Repräsentation des Bereichs der Herzwand durch Ändern der Anzahl von Rasterpositionen, die auf der Basis einer solchen Abnormalitätsbestimmung besetzt sind.

2. Medizinisches Signalverarbeitungssystem (700) nach Anspruch 1, worin die Abnormalitätsbestimmung bestimmt, in welche einer Vielzahl von Kategorien von Abnormalität oder Bereiche von Abnormalitätsausmaß die Messung fällt; und worin die grafische Anzeige (502, 504, 506) der Abnormalität auf die Bereichs- oder Kategoriebestimmung reaktionsfähig ist.

3. Medizinisches Signalverarbeitungssystem (700) nach Anspruch 1 oder 2, worin die grafische Repräsentation auf eine Historie der Abnormalitätsbestimmung reaktionsfähig ist.

4. Medizinisches Signalverarbeitungssystem (700) nach Anspruch 3, worin der Bildschirm (1116) außerdem eine vorherige Abnormalität anzeigt, wenn die Bestimmung normal ist und vorher abnormal war; und/oder worin die grafische Repräsentation eine Dauer einer solchen Abnormalität anzeigt.

5. Medizinisches Signalverarbeitungssystem (700) nach einem vorhergehenden Anspruch, worin die Signaldaten eine Vielzahl von Signalen umfassen, die mit einer Vielzahl der räumlichen Positionen im oder am Organ assoziiert sind, worin die Messung und Bestimmung für jedes der Vielzahl von Signalen ausgeführt werden und worin der Bildschirm (1116) für jedes der Vielzahl von Signalen, reaktionsfähig auf die Messung und Bestimmung, eine Abnormalität an einer solchen räumlichen Position anzeigt, die mit dem Signal assoziiert ist.

6. Medizinisches Signalverarbeitungssystem (700) nach Anspruch 5, worin die grafische Anzeige (502, 504, 506) für ein solches Signal auf eine Abnormalitätsbestimmung für ein oder mehrere andere der Signale reaktionsfähig ist.

7. Medizinisches Signalverarbeitungssystem (700) nach einem vorhergehenden Anspruch, worin die grafische Anzeige (502, 504, 506) eine Repräsentation einer solchen Abnormalität umfasst, deren Größe mit der Dauer und/oder dem Ausmaß der Abnormalität ansteigt.

8. Medizinisches Signalverarbeitungssystem (700) nach einem vorhergehenden Anspruch, worin der Signalprozessor (1110) außerdem dazu konfiguriert ist, für jede einer Vielzahl von Rasterpositionen auf der grafischen Repräsentation des Organs eine solche grafische Anzeige (502, 504, 506) von Abnormalität reaktionsfähig auf die Bestimmung zu bestimmen; und worin vorzugsweise die grafische Anzeige (502, 504, 506) an einer solchen Rasterposition auf eine solche grafische Anzeige (502, 504, 506) an einer oder mehreren benachbarten Positionen reaktionsfähig ist.

9. Medizinisches Signalverarbeitungssystem (700) nach einem vorhergehenden Anspruch, worin die grafische Repräsentation eine Form (502, 504, 506) umfasst, deren Ausdehnung zunimmt, wenn die Abnormalitätsbestimmung fortdauert oder sich verschlimmert; und worin vorzugsweise die Form (502, 504, 506) in Fläche abnimmt, sobald die Abnormalitätsbestimmung endet; worin, wenn eine solche Form in Fläche abnimmt, vorzugsweise eine grafische Repräsentation einer vorherigen Abnormalität zurückgelassen wird.

10. Medizinisches Signalverarbeitungssystem (700) nach einem der Ansprüche 1 bis 9, worin die grafische Repräsentation des Herzens eine oder mehrere 2D-Querschnittansichten des Herzens umfasst und worin die grafische Anzeige (502, 504, 506) einer Abnormalität eine Linie auf einem Querschnitt der Herzwand umfasst; oder worin die grafische Repräsentation des Herzens eine oder mehrere 3D-Ansichten des Herzens umfasst und worin die grafische Anzeige (502, 504, 506) einer Abnormalität einen hervorgehobenen Bereich auf der Herzwand umfasst.

11. Medizinisches Signalverarbeitungssystem (700) nach einem der Ansprüche 1 bis 10, worin der Signalprozessor (1110) außerdem dazu konfiguriert ist, die medizinischen Signaldaten vor dem Ausführen der Messung vorzuverarbeiten, indem ein Maximal- oder Minimalwert eines Signals identifiziert wird, abgeleitet aus dem EKG-Signal innerhalb eines eine Vielzahl von Herzzyklen enthaltenden Zeitfensters;
Identifizieren von Spitzen des abgeleiteten EKG-Signals innerhalb des Zeitfensters durch Bestimmen von anderen Teilen des Signals innerhalb des Zeitfensters innerhalb eines Amplitudenbereichs des Maximal- oder Minimalwerts; und Bestimmen eines mittleren Herzzyklussignals für das Zeitfenster, indem insbesondere Autokorrelationssignale aus der Vielzahl von Herzsignalen gemittelt werden, die unter Verwendung der Spitzen zeitlich ausgerichtet sind.

12. Medizinisches Signalverarbeitungssystem (700) nach Anspruch 1, worin der Prozessor dazu konfiguriert ist, eine fortdauernden Bedingung auf der Basis des einen oder der mehreren EKG-Signale zu bestimmen und, reaktionsfähig auf die Bestimmung der fortdauernden Bedingung, eine solche Veränderung auszuführen.

13. Medizinisches Bilddarstellungssystem, das medizinische Signalverarbeitungssystem (700) nach Anspruch 1 umfassend, wobei das medizinische Bilddarstellungssystem Folgendes umfasst:

ein Bilderfassungssystem (1106, 1114) zum Erfassen eine Bildes eines inneren Organs eines Patienten;
ein medizinisches Signalverarbeitungssystem (1110) zum Erfassen von medizinischen Signaldaten aus dem abgebildeten Organ des Patienten (1104) und zum Verarbeiten der erfassten medizinischen Signaldaten, um eine Abnormalität zu identifizieren; und
ein Bilddarstellungssystem (1116), an das Bilderfassungssystem (1106, 1114) und das medizinische Bildverarbeitungssystem (1110) gekoppelt, um ein Bild des inneren Organs zusammen mit einer grafischen Repräsentation der Abnormalität darzustellen.

14. Medizinisches Bilddarstellungssystem nach Anspruch 13, worin das innere Organ ein menschliches (1104) oder tierisches Herz umfasst und worin die erfassten medizinischen Signaldaten EKG-Daten umfassen, worin insbesondere die Abnormalität eine oder beide von Folgenden umfasst: eine Veränderung im Rhythmus des Herzens und eine ischämische Abnormalität des Herzens.

**15.** Medizinisches Bilddarstellungssystem nach Anspruch 13 oder 14, worin das Bilderfassungssystem ein Röntgenfluoroskopie-Bildgebungssystem umfasst und/oder worin das Bilddarstellungssystem (1116) dazu konfiguriert ist, die grafische Repräsentation darzustellen oder auf dem Bild der Abnormalität auf einer grafischen Repräsentation des inneren Organs darzustellen.

**16.** Medizinisches Bilddarstellungssystem nach einem der Ansprüche 13 bis 15, worin das Bilderfassungssystem (1106, 1114) ein System enthält, um Koordinaten bereitzustellen, die eine erfasste Ansicht des inneren Organs definieren, und worin das Bilddarstellungssystem (1116) auf die Koordinaten reaktionsfähig ist, um die grafische Repräsentation der Abnormalität so anzupassen, dass sie der Ansicht entspricht.

**17.** Medizinisches Bilddarstellungssystem nach einem der Ansprüche 13 bis 16, worin das medizinische Signalverarbeitungssystem (1110) an das Bilderfassungssystem (1106, 1114) gekoppelt ist und dazu konfiguriert ist, die erfassten medizinischen Signaldaten reaktionsfähig auf die Bilddaten für das erfasste Bild zu verarbeiten.

**18.** Medizinisches Bilddarstellungssystem nach Anspruch 17, dazu konfiguriert, einen Bereich des abgebildeten Organs zu identifizieren, auf dem eine medizinische Prozedur ausgeführt wird, und worin das medizinische Signalverarbeitungssystem dazu konfiguriert ist, die Abnormalitätsidentifikation reaktionsfähig auf die Identifikation des Bereichs auszuführen.

**19.** Medizinisches Bilddarstellungssystem nach einem der Ansprüche 13 bis 18, worin das medizinische Signalverarbeitungssystem (1110) einen Signalprozessor umfasst, der konfiguriert ist zum:

Eingeben von medizinischen Signaldaten, die mit einer räumlichen Position im oder am Organ assoziiert sind;
Ausführen von mindestens einer das Organ betreffenden Messung unter Verwendung der medizinischen Signaldaten, um Messdaten bereitzustellen;
aus den Messdaten bestimmen, ob die Messung normal oder abnormal ist; und
Ausgeben von Daten, um auf einer grafischen Repräsentation des Organs eine grafische Anzeige (502, 504, 506) einer Abnormalität an der mit den Signaldaten assoziierten räumlichen Position reaktionsfähig auf Abnormalitätsbestimmung anzuzeigen; worin insbesondere die grafische Repräsentation eine Form umfasst, deren Ausdehnung zunimmt, wenn die Abnormalitätsbestimmung fortdauert oder sich verschlimmert.

**20.** Verfahren zum Darstellen von medizinischen Daten, um Ischämiebeginn eines Herzens anzuzeigen, wobei das Verfahren Folgendes umfasst:

aus einem Bilderfassungssystem (1106, 1114) erfasste Bilddaten eingeben, die ein Bild eines inneren Organs eines Patienten repräsentieren;
Eingeben von medizinischen Signaldaten aus dem inneren Organ des Patienten, wobei die medizinischen Signaldaten das eine oder die mehreren EKG-Signale umfassen, die mit einer räumlichen Position im oder am Herz assoziiert sind;
Verarbeiten der medizinischen Signaldaten, um eine ST-Niveau-Messung zum Identifizieren einer Abnormalität zu bestimmen, wobei ein solches ST-Niveau einer Differenz in der Spannung zwischen zwei Teilen eines EKG-Signals entspricht;
Definieren eines Rasters zum Auflegen auf eine grafische Repräsentation eines Herzens, die einen Bereich einer Wand des Herzens zeigt, wobei der Raster eine Vielzahl von Rasterpositionen umfasst;
Darstellen eines erfassten Bildes des inneren Organs des Patienten zusammen mit einer grafischen Repräsentation der Abnormalität an einer Position auf der grafischen Repräsentation des Herzens, die von der räumlichen Position abgebildet ist;
Variieren der grafischen Repräsentation des Bereichs der Herzwand durch Verändern der Anzahl von Rasterpositionen, die auf der Basis einer solchen Abnormalitätsbestimmung von der grafischen Abnormalitätsanzeige besetzt sind.

**Revendications**

**1.** Système de traitement de signaux médicaux (700) destiné à traiter des données de signaux médicaux obtenues à partir d'un coeur humain (1104) ou d'un coeur animal, et à afficher une représentation graphique des données traitées, en vue de fournir une alerte de crise d'ischémie, le système (700) comprenant :

un module d'entrée de données de signaux médicaux (701) pour entrer un ou plusieurs signaux d'électrocardiogramme ;
un affichage graphique (1116) ; et
un processeur de signaux (1110) couplé audit module d'entrée de données (701) et audit affichage (1116) ;
dans lequel ledit processeur de signaux (1110) est configuré de manière à :

entrer des données de signaux médicaux comprenant ledit un ou lesdits plusieurs signaux d'électrocardiogramme associés à une position spatiale dans ou sur un organe, dans lequel ledit organe est ledit coeur ;
mettre en oeuvre au moins une mesure connexe audit organe en utilisant lesdites données de signaux médicaux, en vue de fournir des données de mesure de niveau de segment ST, un dit niveau de segment ST correspondant à une différence de tension entre deux parties d'un dit signal d'électrocardiogramme ;
déterminer à partir desdits données de mesure si ladite mesure est normale ou anormale ;
définir une grille, à superposer sur une représentation graphique dudit coeur, montrant une zone d'une paroi dudit coeur, ladite grille comprenant une pluralité de positions de grille ;
afficher, à une position sur ladite représentation graphique dudit coeur cartographié à partir de ladite entrée de données de signaux médicaux dudit signal d'électrocardiogramme, une indication graphique (502, 504, 506) d'une anomalie au niveau de ladite position spatiale sur une paroi dudit coeur à laquelle sont associées lesdites données de signal en réponse à une dite détermination d'anomalie ; et
modifier ladite représentation graphique de ladite zone de ladite paroi du coeur en modifiant le nombre desdites positions de grille qui sont occupées sur la base d'une dite détermination d'anomalie.

2. Système de traitement de signaux médicaux (700) selon la revendication 1, dans lequel ladite détermination d'anomalie détermine dans quelle/quel, parmi une pluralité de catégories d'anomalie ou de degrés de sévérité d'anomalie, ladite mesure tombe ; et dans lequel ladite indication graphique (502, 504, 506) de ladite anomalie répond à ladite détermination de catégories ou de degrés de sévérité.

3. Système de traitement de signaux médicaux (700) selon la revendication 1 ou 2, dans lequel ladite représentation graphique répond à un historique de ladite détermination d'anomalie.

4. Système de traitement de signaux médicaux (700) selon la revendication 3, dans lequel ledit affichage (1116) indique en outre une anomalie antérieure lorsque ladite détermination est normale et qu'elle était précédemment anormale, et/ou dans lequel ladite représentation graphique indique une durée d'une dite anomalie.

5. Système de traitement de signaux médicaux (700) selon l'une quelconque des revendications précédentes, dans lequel lesdites données de signal comportent une pluralité de signaux associés à une pluralité desdites positions spatiales dans ou sur ledit organe, dans lequel ladite étape de mesure et ladite étape de détermination sont mises en oeuvre pour chaque signal de ladite pluralité de signaux, et dans lequel ledit affichage (1116) indique graphiquement, pour chaque signal de ladite pluralité de signaux, une anomalie, en réponse à ladite mesure et à ladite détermination, au niveau d'une dite position spatiale associée au signal.

6. Système de traitement de signaux médicaux (700) selon la revendication 5, dans lequel ladite indication graphique (502, 504, 506), pour un dit signal, répond à une détermination d'anomalie pour un ou plusieurs autres desdits signaux.

7. Système de traitement de signaux médicaux (700) selon l'une quelconque des revendications précédentes, dans lequel ladite indication graphique (502, 504, 506) comprend une représentation d'une dite anomalie dont la taille augmente en fonction de la durée et/ou de la gravité de ladite anomalie.

8. Système de traitement de signaux médicaux (700) selon l'une quelconque des revendications précédentes, dans lequel ledit processeur de signaux (1110) est en outre configuré de manière à déterminer, pour chaque position d'une pluralité de positions de grille sur ladite représentation graphique dudit organe, une dite indication graphique (502, 504, 506) d'anomalie en réponse à ladite détermination ; et de préférence, dans lequel, ladite indication graphique (502, 504, 506), au niveau d'une dite position de grille, répond à une dite indication graphique (502, 504, 506) au niveau d'une ou plusieurs positions voisines.

9. Système de traitement de signaux médicaux (700) selon l'une quelconque des revendications précédentes, dans lequel la représentation graphique comporte une forme (502, 504, 506) dont l'étendue augmente à mesure que la détermination d'anomalie persiste ou s'aggrave, et de préférence, dans lequel ladite forme (502, 504, 506) se contracte localement dès lors que la détermination d'anomalie cesse ; et de préférence, dans lequel, lorsqu'une dite forme se contracte localement, une représentation graphique d'une anomalie antérieure est abandonnée.

10. Système de traitement de signaux médicaux (700) selon l'une quelconque des revendications 1 à 9, dans lequel ladite représentation graphique dudit coeur comprend une ou plusieurs vues en coupe transversale 2D dudit coeur, et dans lequel ladite indication graphique (502, 504, 506) d'une anomalie comporte une ligne sur une coupe transversale de ladite paroi du coeur ; ou dans lequel ladite représentation graphique dudit coeur comprend une ou plusieurs vues en 3D dudit coeur, et dans lequel ladite indication graphique (502, 504, 506) d'une anomalie comporte une zone en surbrillance sur ladite paroi du coeur.

11. Système de traitement de signaux médicaux (700) selon l'une quelconque des revendications 1 à 10, dans lequel ledit processeur de signaux (1110) est en outre configuré de manière à prétraiter lesdites données de signaux médicaux avant de mettre en oeuvre ladite mesure, en identifiant une valeur maximale ou minimale d'un signal dérivé dudit signal d'électrocardiogramme dans une fenêtre de temps comprenant une pluralité de cycles cardiaques ;
identifier des crêtes dudit signal d'électrocardiogramme dérivé au sein de ladite fenêtre de temps en déterminant d'autres parties dudit signal au sein de ladite fenêtre de temps dans une plage d'amplitude de ladite valeur maximale ou minimale ;
et déterminer un signal de cycle cardiaque moyen pour ladite fenêtre de temps en calculant la moyenne, en particulier, de signaux d'autocorrélation en provenance de ladite pluralité de signaux cardiaques alignés dans le temps en utilisant lesdites crêtes.

12. Système de traitement de signaux médicaux (700) selon la revendication 1, dans lequel ledit processeur est configuré de manière à déterminer un état persistant sur la base dudit un ou desdits plusieurs signaux d'électrocardiogramme, et à mettre en oeuvre une dite modification en réponse à ladite détermination d'état persistant.

13. Système d'affichage d'images médicales comprenant le système de traitement de signaux médicaux (700) selon la revendication 1, le système d'affichage d'images médicales comprenant :

    un système de capture d'images (1106, 1114) pour capturer une image d'un organe interne d'un patient ;
    un système de traitement de signaux médicaux (1110) pour capturer des données de signaux médicaux à partir de ladite image d'organe dudit patient (1104) et à traiter lesdites données de signaux médicaux capturées en vue d'identifier une anomalie ; et
    un système d'affichage d'images (1116) couplé audit système de capture d'images (1106, 1114) et audit système de traitement d'images médicales (1110), pour afficher une image dudit organe interne avec une représentation graphique de ladite anomalie.

14. Système d'affichage d'images médicales selon la revendication 13, dans lequel ledit organe interne comprend un coeur humain (1104) ou coeur animal, et dans lequel lesdites données de signaux médicaux capturées comportent des données d'électrocardiogramme, en particulier, dans lequel ladite anomalie comporte l'un ou les deux parmi un changement de rythme cardiaque et une anomalie ischémique dudit coeur.

15. Système d'affichage d'images médicales selon la revendication 13 ou 14, dans lequel ledit système de capture d'images comprend un système d'imagerie radioscopique et/ou dans lequel ledit système d'affichage d'images (1116) est configuré de manière à afficher ladite représentation graphique ou ladite image de ladite anomalie sur une représentation graphique dudit organe interne.

16. Système d'affichage d'images médicales selon l'une quelconque des revendications 13 à 15, dans lequel ledit système de capture d'images (1106, 1114) inclut un système permettant de fournir des coordonnées définissant une vue capturée dudit organe interne, et dans lequel ledit système d'affichage d'images (1116) répond auxdites coordonnées pour ajuster ladite représentation graphique de ladite anomalie afin qu'elle corresponde à ladite vue.

17. Système d'affichage d'images médicales selon l'une quelconque des revendications 13 à 16, dans lequel ledit système de traitement de signaux médicaux (1110) est couplé audit système de capture d'images (1106, 1114), et configuré de manière à traiter lesdites données de signaux médicaux capturées en réponse à des données d'image pour ladite image capturée.

18. Système d'affichage d'images médicales selon la revendication 17, configuré de manière à identifier une zone de ladite image d'organe sur laquelle une procédure médicale est mise en oeuvre, et dans lequel ledit système de traitement de signaux médicaux est configuré de manière à mettre en oeuvre ladite identification d'anomalie en réponse à ladite identification de ladite zone.

**19.** Système d'affichage d'images médicales selon l'une quelconque des revendications 13 à 18, dans lequel ledit système de traitement de signaux médicaux (1110) comprend un processeur de signaux configuré de manière à :

entrer des données de signaux médicaux associées à une position spatiale dans ou sur ledit organe ;
mettre en oeuvre au moins une mesure connexe audit organe en utilisant lesdites données de signaux médicaux pour fournir des données de mesure ;
déterminer, à partir desdites données de mesure, si ladite mesure est normale ou anormale ; et
générer en sortie des données pour afficher, sur une représentation graphique dudit organe, une indication graphique (502, 504, 506) d'une anomalie au niveau de ladite position spatiale, à laquelle lesdites données de signal sont associées, en réponse à une dite détermination d'anomalie ; en particulier, dans lequel la représentation graphique comporte une forme dont l'étendue augmente à mesure que la détermination d'anomalie persiste ou s'aggrave.

**20.** Procédé destiné à afficher des données médicales en vue d'indiquer une crise d'ischémie cardiaque, le procédé comprenant les étapes ci-dessous consistant à :

entrer, à partir d'un système de capture d'images (1106, 1114), des données d'image capturées représentant une image d'un organe interne d'un patient ;
entrer les données de signaux médicaux en provenance dudit organe interne dudit patient, lesdites données de signaux médicaux comprenant ledit un ou lesdits plusieurs signaux d'électrocardiogramme associés à une position spatiale dans ou sur ledit coeur ;
traiter lesdites données de signaux médicaux en vue de déterminer une mesure de niveau de segment ST permettant d'identifier une anomalie, un dit niveau de segment ST correspondant à une différence de tension entre deux parties d'un signal d'électrocardiogramme ;
définir une grille, à superposer sur une représentation graphique d'un coeur, montrant une zone d'une paroi dudit coeur, ladite grille comprenant une pluralité de positions de grille ;
afficher une image capturée dudit organe interne dudit patient ainsi qu'une représentation graphique de ladite anomalie à une position sur ladite représentation graphique dudit coeur cartographié à partir de ladite position spatiale ;
modifier ladite représentation graphique de ladite zone de ladite paroi du coeur en modifiant le nombre desdites positions de grille qui sont occupées par ladite indication graphique d'anomalie sur la base d'une dite détermination d'anomalie.

Figure 1

Figure 2

Figure 3

Figure 4a

Figure 4b

Figure 4c

Figure 5

Figure 6

Sensor 1

**701**

Sensor i

Signal
Conditioning,
Filters,
A/D converter

**702**

Optional real time data acquisition system

Keyboard,
Mouse, Data
Input Devices

Video Display

Modem, Fix phone
line or Mobile Phone

storage device -I/O

Audio & Visual
Alarm System

Network , Printer,
Client/Server

Microprocessor System     **700**     Microprocessor

Signal acquisition

Auto-correlation

Data manipulation
and Filtering

Foetal signal
extraction

Diagnostic rules
checker

User Interface and
Operating System

Sensor/lead
mapping; organ
image display

Severity analysis and
graphical abnormality
display

Alarm alerts

Storage and Playback

Program Memory

History Data

Program
Data

Data Storage
Memory

# Figure 7

824 | Signal Acquisition Instrument

800 | Signal Conditioning

823 | Historic Data Stored for offline processing

801 | Band Pass Filtering
(this could be done after the Acquisition Block)

802 | Acquire Signals, Overflow check and Adaptive gain search

803 | Search for Max value (max peak) and Min value (valleys) for each signal within a Time Window (TW)

Change Signal

822

No

804 | Identify all max points (peaks) within the TW which fall within Max – Max * x% (where x is an adaptive value)

Yes

Check if x is above a minimum value

821

805 | Screening of peaks via "soft" time auto-correlation ("soft coherent sequence):
Verify that all peaks (related to the same signal within the TW) have a "soft" time auto correlation. Considering any 3 consecutive peaks (i + 2, i + 1 and i) they must satisfy the following time condition:
ABS(Time_Peak_(i + 2) - Time_Peak_(i + 1) ) -
ABS(Time_Peak_(i + 1) - Time_Peak_(i) ) < Settable Threshold

Reduce value of x

No          820

Yes

806 | Calculate Heart Rate frequency and period on the auto-correlation signal

807 | Perform "smart" average on each signal using as timeframe the period of the Heart Rate.
The timeframe window is "centred" on the time of the peaks max value of the auto-correlation signal.
The values of the overall peaks for each signal are superimposed and averaged.
This is done on all signal.

808 | Baby Signal Extraction Required          Yes
No

810 | Cancel Mother Signal taking away the point to point average signal from the signal within the TW (this for every signal) and repeat

809 | Identify Iso-electric before Q as flat plane between P & Q:
- Search for Q (min before the R peak) on the channel signal used to validate the peaks successfully
- Subtract a programmable delay to the Q time event to get the time & value of the iso-electric

Continue 1

# Figure 8a

Continue 1

811  Identify S point as minimum after peak (R)

812  Add programmable delay to the S time event to get the time & value of the J point

814
Repeat all process with new set of data

813  Calculate ST elevation or depression with reference to the Iso-electric. These ST values (one for each signal) can be "absolute" values or % of the peak values.

# Figure 8a

900 Get new ST values

901 Relate Leads position on the patient (leads' coordinate) to Graphical position on the Heart image file (heart coordinate). This mapping can be changed by the doctor.

902 Assess Gravity applying a pre-defined set of rules

903 Validate the event using an up-down counter (one for each signal) and checking if doctor defined threshold is reached to set or reset the alarm.

904 Define colours and type of message to display based on gravity for each signal. Also based on gravity turn on-off acoustic alarm.

905 Multi Lead 3D Analysis. For each point on the surface of the heart the health condition is determined using a weighted average function based on:
The heart point past health status, the health status of all the point around it, the leads positions (spatial correlation) and ST new values.

906 Associate colour based on the health status. If the status has gone back to normal after an abnormal event then the colour is reset to a default colour different from the "normal" health colour.

907 If required Playback the history of events (fast forward or fast backwards) as it has unfolded showing the evolution of the cardiac event with time.

## Figure 9

1000

Get new ST values

1001 | Relate Leads position on the patient (leads' coordinate) to Graphical position on the Heart image file (heart coordinate). This mapping can be changed by the doctor.

1002 | Assess Gravity applying a pre-defined set of rules

1003 | Validate the event using an up-down counter (one for each signal) and checking if doctor defined threshold is reached to set or reset the alarm.

1004 | Define colours and type of message to display based on gravity for each signal. Also based on gravity turn on-off acoustic alarm.

1005 | Multi Lead 2D Analysis. For each point on the cross sections of the heart the health condition is assessed using a weighted average function based on:
The heart point past health status, the health status of all the point around it, the leads positions (spatial correlation) and ST new values.

1006 | Associate colour based on the health status.- If the status has gone back to normal after an abnormal event then the colour is reset to a default colour different from the "normal" health colour.

1007 | If required Playback the history of events (fast forward or fast backwards) as it has unfolded showing the evolution of the cardiac event with time.

# Figure 10

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 200500389352 A
- US GB94178 A
- US 4846190 A, John
- US 4579125 A, Strobl
- US 4862359 A, Trivedi
- US 6694178 B1, Soula
- JP 05137702 A, Sharp
- EP 1488739 A
- US UPS2003167013 A

### Non-patent literature cited in the description

- **L GEPSTEIN ; S J EVANS.** Electroanatomical mapping of the heart: basic concepts and implications for the treatment of cardiac arrhythmias. *Packing and Clinical Electrophysiology,* June 1998, vol. 21 (6), 1268-1278
- **B TILG et al.** Model-Based Imaging of Cardiac Electrical Excitations in Humans. *IEEE Transactions on Medical Imaging,* September 2002, vol. 21 (9), 1031-1039